(19) Europäisches Patentamt
European Patent Office
Office européen des brevets

(11) Veröffentlichungsnummer: **0 257 296 B1**

# (12) EUROPÄISCHE PATENTSCHRIFT

(45) Veröffentlichungstag der Patentschrift: **12.05.93**

(51) Int. Cl.$^5$: **C07D 231/20**, C07D 231/18, C07D 231/22, C07D 231/26, C07D 401/12, A01N 43/56, A01N 43/40

(21) Anmeldenummer: **87110490.7**

(22) Anmeldetag: **20.07.87**

Die Akte enthält technische Angaben, die nach dem Eingang der Anmeldung eingereicht wurden und die nicht in dieser Patentschrift enthalten sind.

## (54) 4–Cyano(nitro)–5–oxy(thio)–pyrazol–Derivate.

(30) Priorität: **30.07.86 DE 3625686**

(43) Veröffentlichungstag der Anmeldung:
**02.03.88 Patentblatt 88/09**

(45) Bekanntmachung des Hinweises auf die Patenterteilung:
**12.05.93 Patentblatt 93/19**

(84) Benannte Vertragsstaaten:
**AT BE CH DE FR GB IT LI NL**

(56) Entgegenhaltungen:
**US–A– 4 477 462**

(73) Patentinhaber: **BAYER AG**

**W–5090 Leverkusen 1 Bayerwerk(DE)**

(72) Erfinder: **Stetter, Jörg, Dr.**
**Gellertweg 4**
**W–5600 Wuppertal 1(DE)**
Erfinder: **Gehring, Reinhold, Dr.**
**Dasnöckel 49**
**W–5600 Wuppertal 11(DE)**
Erfinder: **Schallner, Otto, Dr.**
**Noldeweg 22**
**W–4019 Monheim(DE)**
Erfinder: **Santel, Hans–Joachim, Dr.**
**Grünstrasse 9a**
**W–5090 Leverkusen 1(DE)**
Erfinder: **Schmidt, Robert R., Dr.**
**Im Waldwinkel 110**
**W–5060 Bergisch–Gladbach 2(DE)**
Erfinder: **Lürssen, Klaus, Dr.**
**August–Kierspel–Strasse 151**
**W–5060 Bergisch–Gladbach 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 257 296 B1

## Beschreibung

Die Erfindung betrifft neue 4 – Cyano(nitro) – 5 – oxy(thio)pyrazol – Derivate,mehrere Verfahren zu ihrer Herstellung sowie ihre Verwendung als Herbizide und Wachstumsregulatoren.

Es ist bereits bekannt, daß bestimmte 5 – Acylamido – 1 – aryl – pyrazole, wie beispielsweise das 4 – Cyano – 5 – propionamido – 1 – (2,4,6 – trichlorphenyl) – pyrazol herbizide Eigenschaften besitzen (vgl. z.B. DE – OS 32 26 513).

Die herbizide Wirksamkeit dieser vorbekannten Verbindungen gegenüber Unkräutern ist jedoch ebenso wie ihre Verträglichkeit gegenüber wichtigen Kulturpflanzen nicht immer in allen Anwendungsbereichen völlig zufriedenstellend.

Es wurden neue 4 – Cyano(nitro) – 5 – oxy(thio) – pyrazol – Derivate der Formel

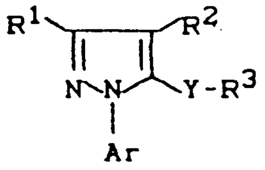 (I)

in welcher

$R^1$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^2$ für Nitro oder Cyano steht,

$R^3$ für jeweils gegebenenfalls substituiertes, geradkettiges oder verzweigtes Alkyl, Alkenyl und Alkinyl mit jeweils bis zu 6 Kohlenstoffatomen steht, wobei als Substituenten jeweils genannt seien:
Halogen; Alkoxy, Alkylthio, Alkylsulfinyl, Alkylsulfonyl und Dialkoxy mit jeweils 1 bis 4 Kohlenstoffatomen in den Alkylteilen; Cyano; Hydroxycarbonyl; Alkoxycarbonyl, Alkylthiocarbonyl und Alkylcarbonyl mit jeweils 1 bis 4 Kohlenstoffatomen im Alkylteil; Aminocarbonyl; Alkylaminocarbonyl und Dialkylaminocarbonyl mit jeweils 1 bis 4 Kohlenstoffatomen in den Alkylteilen; Hydroximino und Alkoximino mit 1 bis 4 Kohlenstoffatomen;
sowie die Gruppierung

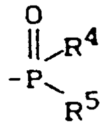 ,

wobei

$R^4$ und $R^5$ gleich oder verschieden sind und für Hydroxy, Alkyl und Alkoxy mit jeweils 1 bis 4 Kohlenstoffatomen, sowie für jeweils gegebenenfalls einfach oder mehrfach, gleich oder verschieden substituiertes Phenyl und Phenoxy stehen, wobei als Substituenten vorzugsweise die für Ar als Phenylsubstituenten infrage kommenden Reste genannt seien;
ferner für jeweils gegebenenfalls einfach oder mehrfach, gleich oder verschieden substituiertes Cycloalkyl und Cycloalkylalkyl mit 3 bis 7 Kohlenstoffatomen im Cycloalkylteil und 1 bis 2 Kohlenstoffatomen im Alkylteil steht, wobei als Substituenten im Cycloalkylteil jeweils genannt seien: Halogen, Alkyl, Alkoxy und Alkylthio mit jeweils 1 bis 4 Kohlenstoffatomen;
oder für jeweils gegebenenfalls einfach oder mehrfach, gleich oder verschieden substituiertes Phenyl und Phenylalkyl mit 1 bis 4 Kohlenstoffatomen im geradkettigen oder verzweigten Alkylteil steht, wobei als Phenylsubstituenten jeweils infrage kommen:
Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen;

Y für O, S, SO oder $SO_2$ steht und

Ar für zweifach oder höher substituiertes Phenyl sowie für jeweils gegebenenfalls einfach oder mehrfach, gleich oder verschieden substituiertes 2-Pyridyl, 3-Pyridyl oder 4-Pyridyl steht, wobei als Phenyl- bzw. Pyridyl-Substituenten in Frage kommen:
Cyano, Nitro, Halogen, jeweils geradkettiges oder verzweigtes Alkyl, Alkoxy oder Alkoxycarbonyl mit jeweils bis zu 4 Kohlenstoffatomen außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_mR^6$
wobei

$R^6$ für Amine, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für eine Zahl 0, 1 oder 2 steht.

Weiterhin wurde gefunden, daß man die neuen 4-Cyano(nitro)-5-oxy(thio)-pyrazol-Derivate der allgemeinen Formel (I)

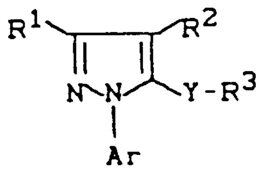

(I)

in welcher

$R^1$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^2$ für Nitro oder Cyano steht,

$R^3$ für jeweils gegebenenfalls substituiertes, geradkettiges oder verzweigtes Alkyl, Alkenyl und Alkinyl mit jeweils bis zu 6 Kohlenstoffatomen steht, wobei als Substituenten jeweils genannt seien:
Halogen; Alkoxy, Alkylthio, Alkylsulfinyl, Alkylsulfonyl und Dialkoxy mit jeweils 1 bis 4 Kohlenstoffatomen in den Alkylteilen; Cyano; Hydroxycarbonyl; Alkoxycarbonyl, Alkylthiocarbonyl und Alkylcarbonyl mit jeweils 1 bis 4 Kohlenstoffatomen im Alkylteil; Aminocarbonyl; Alkylaminocarbonyl und Dialkylaminocarbonyl mit jeweils 1 bis 4 Kohlenstoffatomen in den Alkylteilen; Hydroximino und Alkoximino mit 1 bis 4 Kohlenstoffatomen;
sowie die Gruppierung

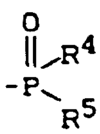 ,

wobei

$R^4$ und $R^5$ gleich oder verschieden sind und für Hydroxy, Alkyl und Alkoxy mit jeweils 1 bis 4 Kohlenstoffatomen, sowie für jeweils gegebenenfalls einfach oder mehrfach, gleich oder verschieden substituiertes Phenyl und Phenoxy stehen, wobei als Substituenten vorzugsweise die für Ar als Phenylsubstituenten infrage kommenden Reste genannt seien;
ferner für jeweils gegebenenfalls einfach oder mehrfach, gleich oder verschieden substituiertes Cycloalkyl und Cycloalkylalkyl mit 3 bis 7 Kohlenstoffatomen im Cycloalkylteil und 1 bis 2 Kohlenstoffatomen im Alkylteil steht, wobei als Substituenten im Cycloalkylteil jeweils genannt seien: Halogen, Alkyl, Alkoxy und Alkylthio mit jeweils 1 bis 4 Kohlenstoffatomen;
oder für jeweils gegebenenfalls einfach oder mehrfach, gleich oder verschieden substituiertes Phenyl und Phenylalkyl mit 1 bis 4 Kohlenstoffatomen im geradkettigen oder verzweigten Alkylteil steht, wobei als Phenylsubstituenten jeweils infrage kommen:
Halogen, Cyano, Nitro, jeweils geradkettiges oder verzweigtes Alkyl, Alkoxy, Alkylthio,

Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen;

Y für O, S, SO oder $SO_2$ steht und

Ar für zweifach oder höher substituiertes Phenyl sowie für jeweils gegebenenfalls einfach oder mehrfach, gleich oder verschieden substituiertes 2-Pyridyl, 3-Pyridyl oder 4-Pyridyl steht, wobei als Phenyl- bzw. Pyridyl-Substituenten in Frage kommen:
Cyano, Nitro, Halogen, jeweils geradkettiges oder verzweigtes Alkyl, Alkoxy oder Alkoxycarbonyl mit jeweils bis zu 4 Kohlenstoffatomen außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_mR^6$
wobei

$R^6$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für eine Zahl 0, 1 oder 2 steht,

mit Hilfe eines der im folgenden beschriebenen Herstellungsverfahren erhält.

a) Man erhält 4-Cyano(nitro)-5-oxy(thio)-pyrazol-Derivate der Formel (I)

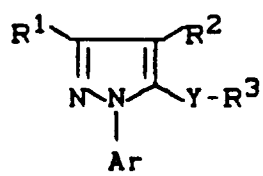

(I)

in welcher

$R^1$, $R^2$, $R^3$, Y und Ar die oben angegebene Bedeutung haben,

wenn man 5-Halogen-pyrazol-Derivate der Formel (II)

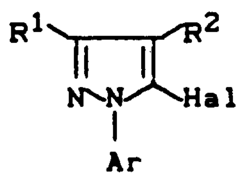

(II)

in welcher

$R^1$, $R^2$ und Ar die oben angegebene Bedeutung haben und

Hal für Fluor, Chlor oder Brom steht,

mit nucleophilen Verbindungen der Formel (III)

$H - Y - R^3$ (III)

in welcher

$R^3$ und Y die oben angegebene Bedeutung haben,

in Gegenwart eines Verdünnungsmittels und gegebenenfalls in Gegenwart eines Säurebindemittels umsetzt.

b) Man erhält 4-Nitro-5-oxy(thio)-pyrazol-Derivate der Formel (Ia)

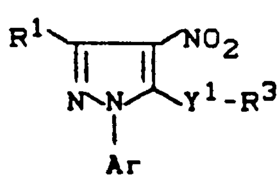

(Ia)

in welcher

$R^1$, $R^3$ und Ar die oben angegebene Bedeutung haben und

$Y^1$ für Sauerstoff oder Schwefel steht,

wenn man 5 – Oxy(thio) – pyrazol – Derivate der Formel (IV)

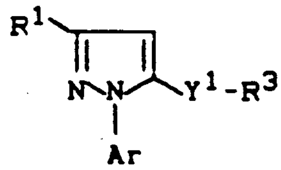

(IV)

in welcher

$R^1$, $R^3$, $Y^1$ und Ar die oben angegebene Bedeutung haben,

mit einem Nitrierungsmittel gegebenenfalls in Gegenwart eines Verdünnungsmittels und gegebenenfalls in Gegenwart eines Katalysators umsetzt.

c) Man erhält 4 – Cyano(nitro) – 5 – oxy(thio) – pyrazol – Derivate der Formel (Ib)

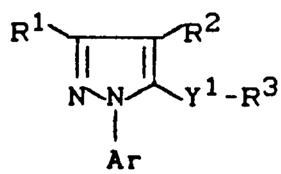

(Ib)

in welcher

$R^1$, $R^2$, $R^3$, $Y^1$ und Ar die oben angegebene Bedeutung haben,

wenn man Pyrazolin – one(thione) der Formel (V)

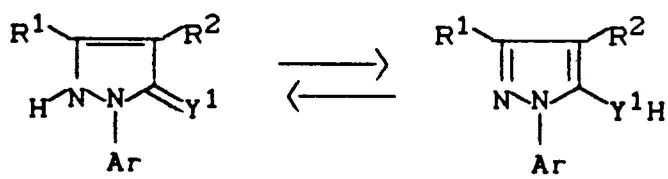

(V)

in welcher

$R^1$, $R^2$, Ar und $Y^1$ die oben angegebene Bedeutung haben,

mit Alkylierungsmitteln der Formel (VI)

$A - R^3$ (VI)

worin

$R^3$ die oben angegebene Bedeutung hat und

A für eine elektronenziehende Abgangsgruppe steht,

gegebenenfalls in Gegenwart eines Verdünnungsmittels und gegebenenfalls in Gegenwart eines Säurebindemittels umsetzt, wobei je nach Reaktionsbedingungen auch die entsprechenden N – alkylierten Derivate der Formel (VII)

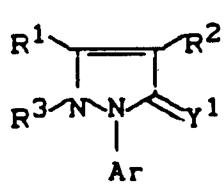

(VII)

in welcher
$R^1$, $R^2$, $R^3$, Ar und $Y^1$ die oben angegebene Bedeutung haben,
erhalten werden können.
d) Man erhält 5-Sulfinyl(sulfonyl)-pyrazol-Derivate der Formel (Ic)

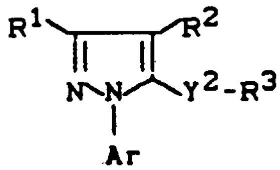
(Ic)

in welcher
$R^1$, $R^2$, $R^3$ und Ar die oben angegebene Bedeutung haben und
$Y^2$ für SO oder $SO_2$ steht,
wenn man 5-Thio-pyrazol-Derivate der Formel (Id)

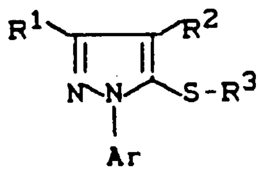
(Id)

in welcher
$R^1$, $R^2$, $R^3$ und Ar die oben angegebene Bedeutung haben,
nach bekannten Methoden in üblicher Weise oxidiert.
e) Man erhält 4-Cyano(nitro)-5-oxy(thio)-pyrazol-Derivate der Formel (Ib)

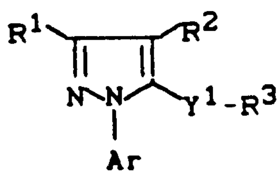
(Ib)

in welcher
$R^1$, $R^2$, $R^3$, $Y^1$ und Ar die oben angegebene Bedeutung haben,
wenn man 5-Sulfonyl-pyrazol-Derivate der Formel (Ie)

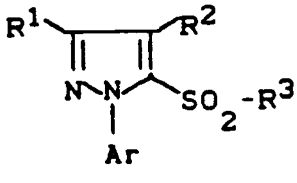
(Ie)

in welcher
$R^1$, $R^2$, $R^3$ und Ar die oben angegebene Bedeutung haben,
mit nucleophilen Verbindungen der Formel (IIIa)

$$H - Y^1 - R^3 \quad \text{(IIIa)}$$

in welcher
$R^3$ und $Y^1$ die oben angegebene Bedeutung haben,

in Gegenwart eines Verdünnungsmittels und gegebenenfalls in Gegenwart eines Säurebindemittels umsetzt.

Schließlich wurde gefunden, daß die neuen 4-Cyano(nitro)-5-oxy(thio)-pyrazol-Derivate der allgemeinen Formel (I) herbizide, insbesondere auch selektiv-herbizide, sowie wachstumsregulierende Eigenschaften besitzen.

Überraschenderweise zeigen die erfindungsgemäßen 4-Cyano(nitro)-5-oxy(thio)-pyrazol-Derivate der allgemeinen Formel (I) eine erheblich bessere allgemein-herbizide Wirksamkeit gegen schwer bekämpfbare Problemunkräuter und gleichzeitig eine deutlich verbesserte Verträglichkeit gegenüber wichtigen Kulturpflanzen im Vergleich zu den aus dem Stand der Technik bekannten 5-Acylamido-1-arylpyrazolen, wie beispielsweise dem 4-Cyano-5-propionamido-1-(2,4,6-trichlorphenyl)-pyrazol, welche chemisch und wirkungsmäßig naheliegende Verbindungen sind.

Die erfindungsgemäßen 4-Cyano(nitro)-5-oxy(thio)-pyrazol-Derivate sind durch die Formel (I) allgemein definiert. Bevorzugt sind diejenigen Verbindungen der Formel (I), bei welchen

$R^1$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^2$ für Nitro oder Cyano steht,

$R^3$ für jeweils gegebenenfalls substituiertes, geradkettiges oder verzweigtes Alkyl, Alkenyl und Alkinyl mit jeweils bis zu 6 Kohlenstoffatomen steht, wobei als Substituenten jeweils genannt seien:
Halogen; Alkoxy, Alkylthio, Alkylsulfinyl, Alkylsulfonyl und Dialkoxy mit jeweils 1 bis 4 Kohlenstoffatomen in den Alkylteilen; Cyano; Hydroxycarbonyl; Alkoxycarbonyl, Alkylthiocarbonyl und Alkylcarbonyl mit jeweils 1 bis 4 Kohlenstoffatomen im Alkylteil; Aminocarbonyl; Alkylaminocarbonyl und Dialkylaminocarbonyl mit jeweils 1 bis 4 Kohlenstoffatomen in den Alkylteilen; Hydroximino und Alkoximino mit 1 bis 4 Kohlenstoffatomen;
sowie die Gruppierung

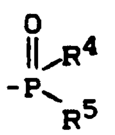 ,

wobei

$R^4$ und $R^5$ gleich oder verschieden sind und für Hydroxy, Alkyl und Alkoxy mit jeweils 1 bis 4 Kohlenstoffatomen, sowie für jeweils gegebenenfalls einfach oder mehrfach, gleich oder verschieden substituiertes Phenyl und Phenoxy stehen, wobei als Substituenten vorzugsweise die für Ar als Phenylsubstituenten infrage kommenden Reste genannt seien;
ferner für jeweils gegebenenfalls einfach oder mehrfach, gleich oder verschieden substituiertes Cycloalkyl und Cycloalkylalkyl mit 3 bis 7 Kohlenstoffatomen im Cycloalkylteil und 1 bis 2 Kohlenstoffatomen im Alkylteil steht, wobei als Substituenten im Cycloalkylteil jeweils genannt seien: Halogen, Alkyl, Alkoxy und Alkylthio mit jeweils 1 bis 4 Kohlenstoffatomen;
oder für jeweils gegebenenfalls einfach oder mehrfach, gleich oder verschieden substituiertes Phenyl und Phenylalkyl mit 1 bis 4 Kohlenstoffatomen im geradkettigen oder verzweigten Alkylteil steht, wobei als Phenylsubstituenten jeweils infrage kommen:
Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen;

Y für O, S, SO oder $SO_2$ steht und

Ar für zweifach oder höher substituiertes Phenyl sowie für jeweils gegebenenfalls einfach oder mehrfach, gleich oder verschieden substituiertes 2-Pyridyl, 3-Pyridyl oder 4-Pyridyl steht, wobei als Phenyl- bzw. Pyridyl-Substituenten in Frage kommen:
Cyano, Nitro, Halogen, jeweils geradkettiges oder verzweigtes Alkyl, Alkoxy oder Alkoxycarbonyl mit jeweils bis zu 4 Kohlenstoffatomen außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_mR^6$

wobei

$R^6$ für Amine, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für eine Zahl 0, 1 oder 2 steht.

Bevorzugt sind diejenigen Verbindungen der Formel (I), bei welchen

$R^1$ für Wasserstoff, Methyl, Ethyl, n- oder i-Propyl und Trifluormethyl steht;

$R^2$ für Nitro oder Cyano steht;

$R^3$ für Methyl, Ethyl; n- oder i-Propyl; n-, i-, s- oder t-Butyl; n- oder i-Pentyl; n- oder i-Hexyl, Allyl, Propenyl, Butenyl, Propargyl oder Butinyl steht, die jeweils einfach oder mehrfach, gleich oder verschieden substituiert sein können durch Fluor, Chlor, Brom, Methoxy, Ethoxy, Methylthio, Ethylthio, Methylsulfinyl, Methylsulfonyl, Dimethoxy, Diethoxy, Cyano, Hydroxycarbonyl, Methoxycarbonyl, Ethoxycarbonyl, Propoxycarbonyl, Butoxycarbonyl, Aminocarbonyl, Methylaminocarbonyl, Dimethylaminocarbonyl, Methylthiocarbonyl, Methylcarbonyl, Hydroximino, Methoximino, Ethoximino, Phosphonoyl, Methyl-phosphinoyl, Dimethylphosphinoyl, Methylethylphosphinoyl, Dimethylphosphonoyl sowie Diethylphosphonoyl;
ferner für Cyclopropyl, Cyclopentyl, Cyclohexyl, Cyclopropylmethyl, Cyclopentylmethyl oder Cyclohexylmethyl steht, die jeweils einfach oder zweifach, gleich oder verschieden durch Fluor, Chlor oder Methyl substituiert sein können;
oder für jeweils gegebenenfalls ein- bis dreifach, gleich oder verschieden substituiertes Phenyl, Benzyl oder Phenylethyl steht, wobei als Phenylsubstituenten in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl und Trifluormethylsulfonyl;

Y für O, S, SO oder $SO_2$ steht und

Ar für zweifach oder höher substituiertes Phenyl sowie für jeweils gegebenenfalls ein- bis vierfach, gleich oder verschieden substituiertes 2-Pyridyl, 3-Pyridyl oder 4-Pyridyl steht, wobei als Phenyl- bzw. Pyridyl-Substituenten in Frage kommen:
Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Difluorchlormethyl, Dichlorfluormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_mR^6$,
wobei

$R^6$ für Amine, Methylamino, Ethylamino, Dimethylamino, Diethylamino, Fluordichlormethyl, Difluormethyl, Tetrafluorethyl, Trifluorchlerethyl, Trifluormethyl, Methyl oder Ethyl steht und

m für eine Zahl 0, 1 oder 2 steht.

Insbesondere sind die Verbindungen der Formel (I) hervorzuheben, in denen Y für Sauerstoff oder Schwefel steht und $R^1$, $R^2$, $R^3$ und Ar die oben als besonders bevorzugt angegebene Bedeutung haben.

Im einzelnen seien außer den bei den Herstellungsbeispielen genannten Verbindungen die in der folgenden Tabelle formelmäßig aufgeführten 4-Cyano(nitro)-5-oxy(thio)-pyrazol-Derivateder allgemeinen Formel (I) genannt:

**Tabelle 1**

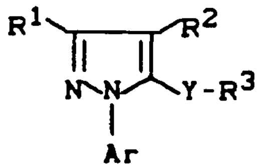 (I)

| $R^1$ | $R^2$ | $R^3$ | Y | Ar |
|---|---|---|---|---|
| H | $NO_2$ | $-CH_2-COOH$ | O | 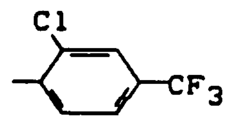 |
| H | $NO_2$ | $-CH_2-COOCH_3$ | O | 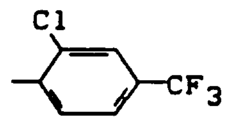 |
| H | $NO_2$ | $-CH_2-COOC_2H_5$ | O | 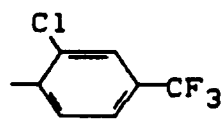 |
| H | $NO_2$ | $-CH_2CN$ | O | 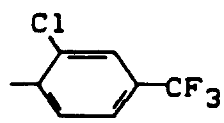 |
| H | $NO_2$ | 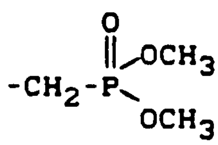 | O | 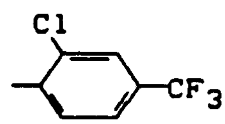 |
| H | $NO_2$ | 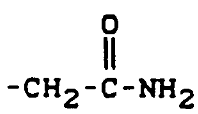 | O | 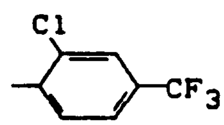 |
| H | $NO_2$ | 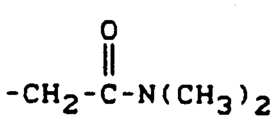 | O | 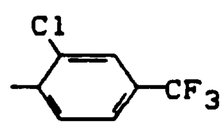 |

Tabelle 1 (Fortsetzung)

| $R^1$ | $R^2$ | $R^3$ | Y | Ar |
|---|---|---|---|---|
| H | $NO_2$ | $-CH_2-CH=CH_2$ | O | 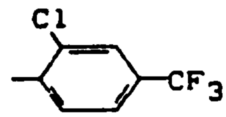 |
| H | $NO_2$ | $-CH_2-C\equiv CH$ | O | 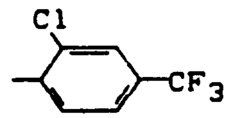 |
| H | $NO_2$ | $-CH_2CH_2CN$ | O | 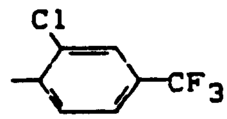 |
| H | $NO_2$ | $-CH_2CH_2\overset{\overset{O}{\|}}{C}-OCH_3$ | O | 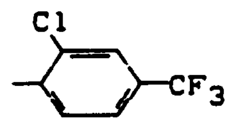 |
| H | $NO_2$ | $-CH_2CH_2OCH_3$ | O | 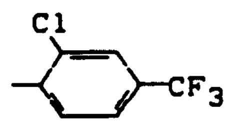 |
| H | $NO_2$ | $-CH_2$ 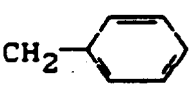 | O | 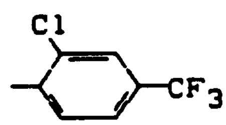 |
| H | $NO_2$ | $-CH_2-CH=N-OCH_3$ | O | 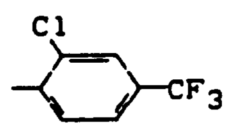 |
| H | $NO_2$ | $-CH_2-\underset{\underset{CH_3}{\|}}{C}=N-OCH_3$ | O | 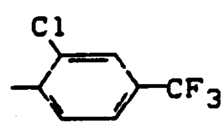 |

Tabelle 1 (Fortsetzung)

| $R^1$ | $R^2$ | $R^3$ | Y | Ar |
|---|---|---|---|---|
| H | CN | $-C_2H_5$ | O | 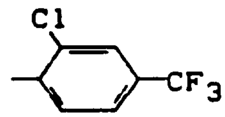 |
| H | CN | $-C_3H_7$ | O | 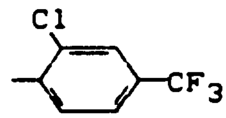 |
| H | CN | $CH_3$<br>$-CH-COOC_4H_9$ | O | 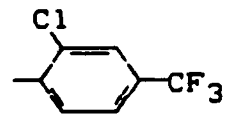 |
| H | CN | $-CH_2CN$ | O | 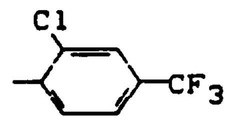 |
| H | CN | $-CH_2-COOCH_3$ | O | 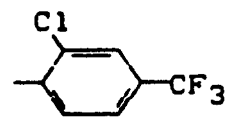 |
| H | CN | $-CH_2-CONH_2$ | O | 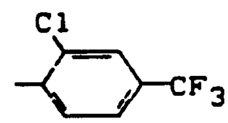 |
| H | CN | $-CH_2-CO-N(CH_3)_2$ | O | 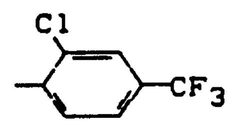 |
| H | CN | $CH_3$<br>$-CH-CO-NH_2$ | O | 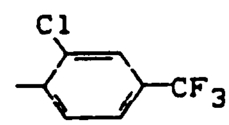 |

Tabelle 1 (Fortsetzung)

| $R^1$ | $R^2$ | $R^3$ | Y | Ar |
|---|---|---|---|---|
| H | CN | $CH_3$<br>\|<br>$-CH-CO-N(CH_3)_2$ | O | 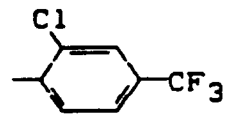 |
| H | CN | O<br>‖ $OCH_3$<br>$-CH_2-P$<br>$OCH_3$ | O | 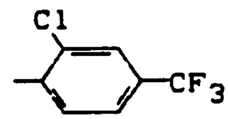 |
| H | CN | O<br>‖ $OC_2H_5$<br>$-CH_2-P$<br>$OC_2H_5$ | O | 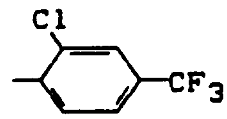 |
| H | $NO_2$ | $-CH_2-COOCH_3$ | O | 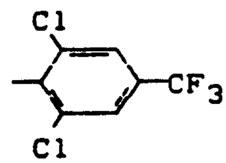 |
| H | $NO_2$ | $-CH_2-COOC_4H_9$ | O | 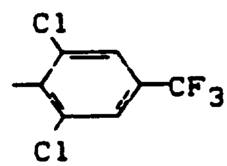 |
| H | $NO_2$ | $CH_3$<br>\|<br>$-CH-CO-NH_2$ | O | 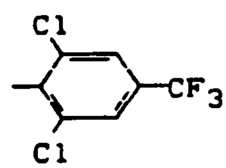 |
| H | $NO_2$ | $CH_3$<br>\|<br>$-CH-COOH$ | O | 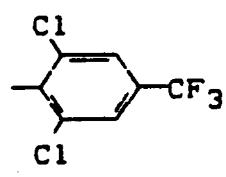 |

Tabelle 1 (Fortsetzung)

| $R^1$ | $R^2$ | $R^3$ | Y | Ar |
|---|---|---|---|---|
| H | $NO_2$ | 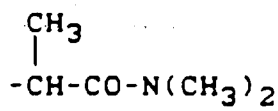 | O | 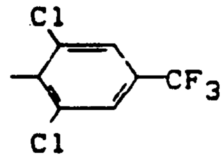 |
| H | $NO_2$ | $-CH_2CN$ | O | 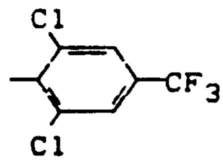 |
| H | $NO_2$ | 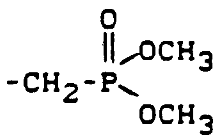 | O | 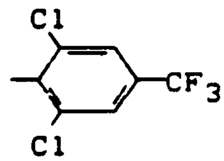 |
| H | $NO_2$ | 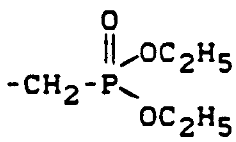 | O | 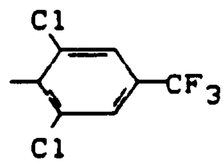 |
| H | $NO_2$ | 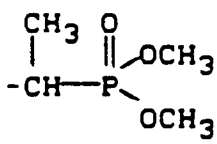 | O | 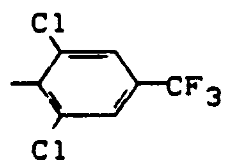 |
| H | $NO_2$ | 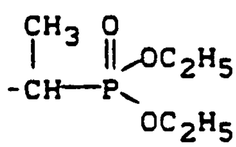 | O | 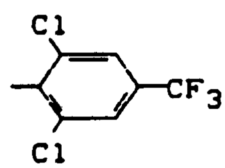 |
| H | $NO_2$ | $-CH_2CH_2-COOCH_3$ | O | 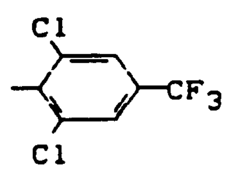 |

Tabelle 1 (Fortsetzung)

| $R^1$ | $R^2$ | $R^3$ | Y | Ar |
|---|---|---|---|---|
| H | $NO_2$ | $-CH_2CH_2CN$ | O | 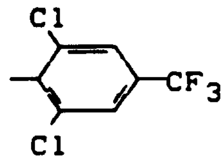 |
| H | $NO_2$ | $-CH_2-C{\equiv}CH$ | O | 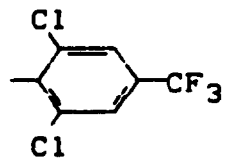 |
| H | $NO_2$ | $-CH_2-CH{=}N-OCH_3$ | O | 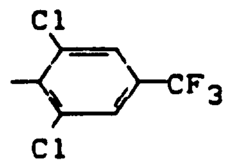 |
| H | $NO_2$ | $-CH_2-C(CH_3){=}N-OCH_3$ | O | 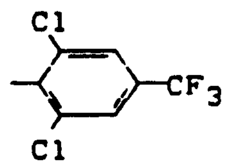 |
| H | $NO_2$ | $-CH_2CH_2OCH_3$ | O | 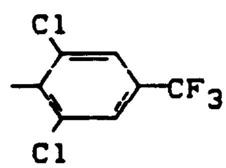 |
| H | CN | $-CH_2-COOCH_3$ | O | 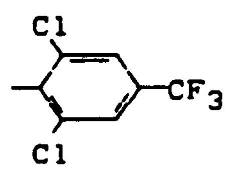 |
| H | CN | $-CH_2-C(=O)-NH_2$ | O | 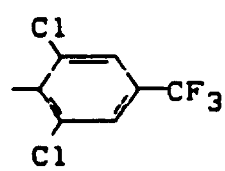 |

Tabelle 1 (Fortsetzung)

| $R^1$ | $R^2$ | $R^3$ | Y | Ar |
|---|---|---|---|---|
| H | CN | 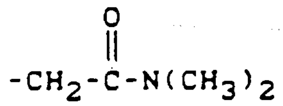 | O | 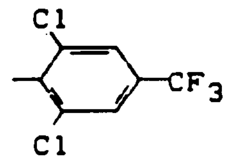 |
| H | CN | 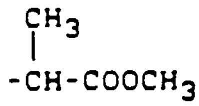 | O | 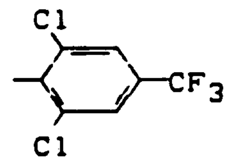 |
| H | CN | 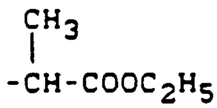 | O | 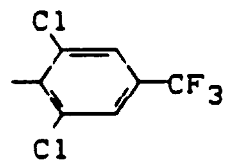 |
| H | CN | 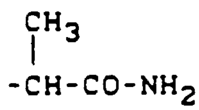 | O | 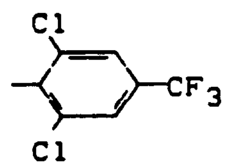 |
| H | CN | 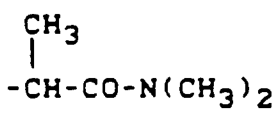 | O | 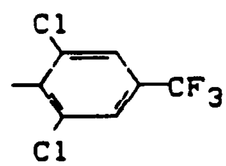 |
| H | CN | $-CH_2CH_2OCH_3$ | O | 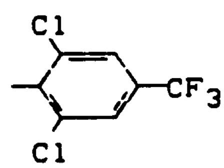 |
| H | CN | $-CH_2CH_2COOCH_3$ | O | 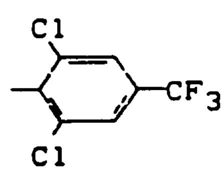 |

Tabelle 1 (Fortsetzung)

| $R^1$ | $R^2$ | $R^3$ | Y | Ar |
|---|---|---|---|---|
| H | CN | $-CH_2-CH_2COOC_4H_9$ | O | 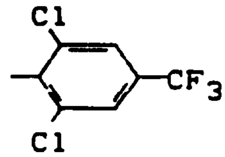 |
| H | $NO_2$ | $-CH_2-COOCH_3$ | O | 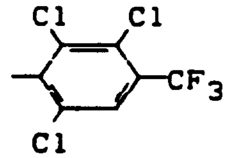 |
| H | $NO_2$ | $-CH(CH_3)-COOCH_3$ | O | 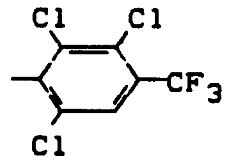 |
| H | $NO_2$ | $-CH_2COOC_2H_5$ | O | 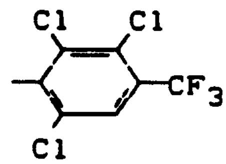 |
| H | $NO_2$ | $-CH(CH_3)-COOC_2H_5$ | O | 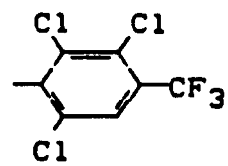 |
| H | $NO_2$ | $-CH_2-COOC_4H_9$ | O | 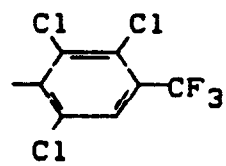 |
| H | $NO_2$ | $-CH_2-COOH$ | O | 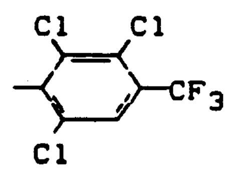 |

Tabelle 1 (Fortsetzung)

| $R^1$ | $R^2$ | $R^3$ | Y | Ar |
|---|---|---|---|---|
| H | $NO_2$ | 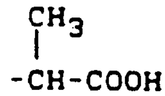 | O | 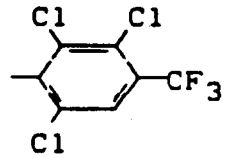 |
| H | $NO_2$ | 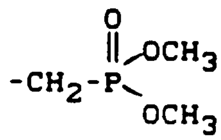 | O | 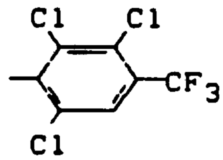 |
| H | $NO_2$ | 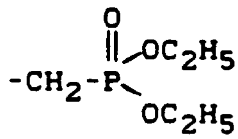 | O | 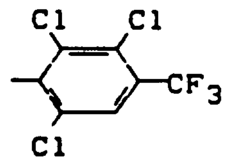 |
| H | $NO_2$ | $-CH_2CN$ | O | 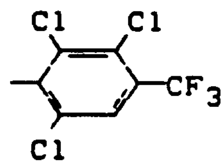 |
| H | $NO_2$ | $-CH_2CH_2OCH_3$ | O | 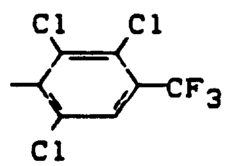 |
| H | $NO_2$ | $-CH_3$ | O | 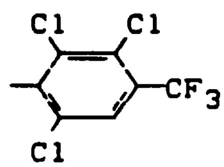 |
| H | $NO_2$ | $-C_2H_5$ | O | 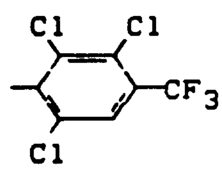 |

Tabelle 1 (Fortsetzung)

| $R^1$ | $R^2$ | $R^3$ | Y | Ar |
|---|---|---|---|---|
| H | $NO_2$ | $-C_3H_7$ | O | 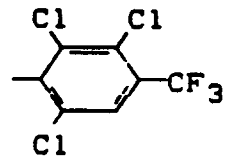 |
| H | $NO_2$ | $-CH_2CH=CH_2$ | O | 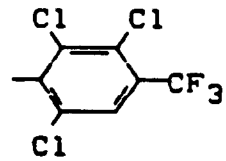 |
| H | $NO_2$ | $-CH_2-CH=N-OCH_3$ | O | 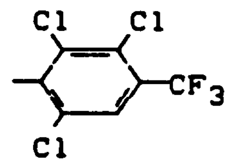 |
| H | CN | $-CH_2-COOCH_3$ | O | 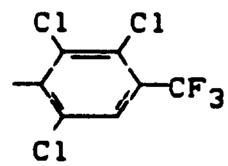 |
| H | CN | $CH_3$<br>│<br>$-CH-COOCH_3$ | O | 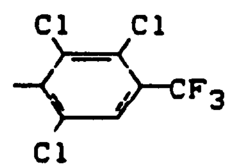 |
| H | CN | $-CH_2-COOC_2H_5$ | O | 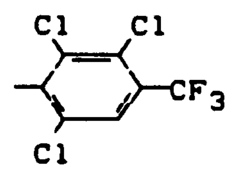 |
| H | CN | $CH_3$<br>│<br>$-CH-COOC_2H_5$ | O | 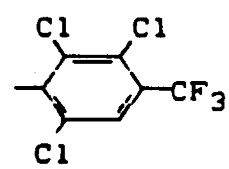 |

Tabelle 1 (Fortsetzung)

| $R^1$ | $R^2$ | $R^3$ | Y | Ar |
|---|---|---|---|---|
| H | CN | $-CH_2-COOH$ | O | 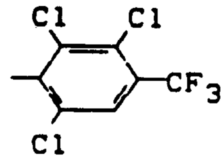 |
| H | CN | 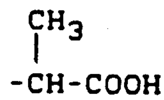 | O | 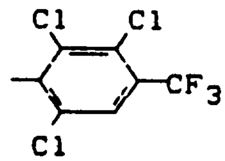 |
| H | CN | 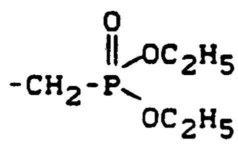 | O | 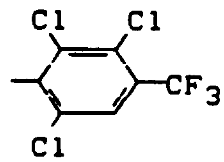 |
| H | CN | $-CH_3$ | O | 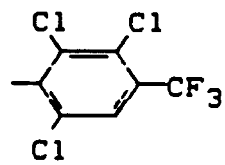 |
| H | CN | $-C_2H_5$ | O | 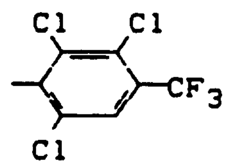 |
| H | CN | $-CH_2CN$ | O | 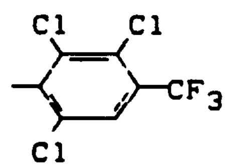 |
| H | $NO_2$ | $-CH_3$ | O | 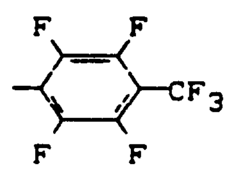 |

Tabelle 1 (Fortsetzung)

| $R^1$ | $R^2$ | $R^3$ | Y | Ar |
|---|---|---|---|---|
| H | $-NO_2$ | $-C_2H_5$ | O | 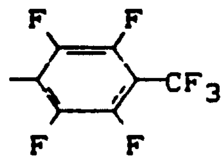 |
| H | $NO_2$ | $-CH_2-COOH$ | O | 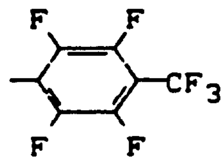 |
| H | $NO_2$ | $-CH_2-COOCH_3$ | O | 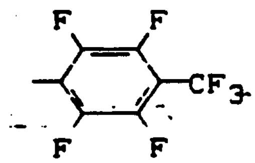 |
| H | $NO_2$ | $-CH(CH_3)-COOH$ | O | 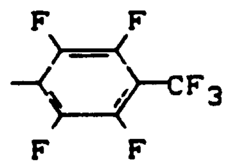 |
| H | $NO_2$ | $-CH(CH_3)-COOC_4H_9$ | O | 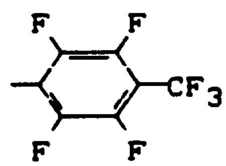 |
| H | $NO_2$ | $-CH(CH_3)-COOCH_3$ | O | 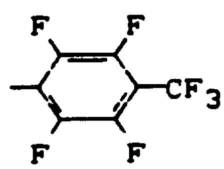 |
| H | $NO_2$ | $-CH_2CN$ | O | 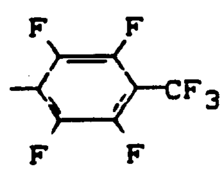 |

Tabelle 1 (Fortsetzung)

| $R^1$ | $R^2$ | $R^3$ | Y | Ar |
|---|---|---|---|---|
| H | $NO_2$ | 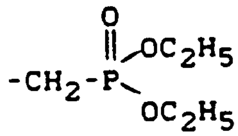 | O | 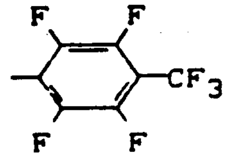 |
| H | $NO_2$ | $-CH_2-CH=CH_2$ | O | 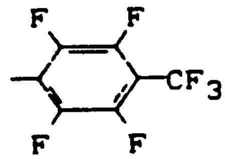 |
| H | $NO_2$ | $-CH_2-C\equiv CH$ | O | 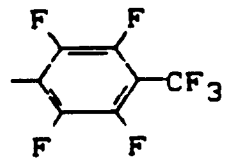 |
| H | CN | $-CH_3$ | O | 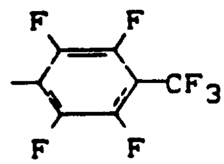 |
| H | CN | $-C_2H_5$ | O | 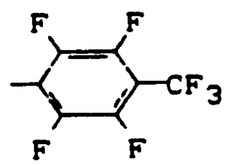 |
| H | CN | $-CH_2-COOH$ | O | 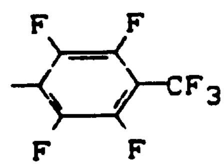 |
| H | CN | 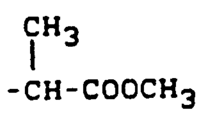 | O | 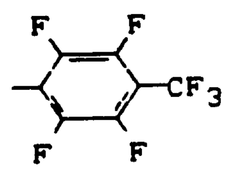 |

Tabelle 1 (Fortsetzung)

| $R^1$ | $R^2$ | $R^3$ | Y | Ar |
|---|---|---|---|---|
| H | CN | $CH_3$<br>\|<br>$-CH-COOC_2H_5$ | O | 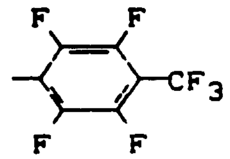 |
| H | CN | $CH_3$<br>\|<br>$-CH-COOC_4H_9$ | O | 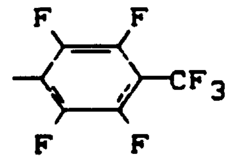 |
| H | CN | $-CH_2-COOCH_3$ | O | 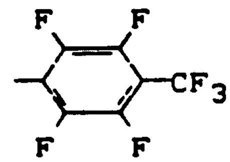 |

Verwendet man beispielsweise 5-Chlor-1-(2,6-dichlor-4-trifluormethyl-phenyl)-4-nitro-pyrazol und Propanol als Ausgangsstoffe, so läßt sich der Reaktionsablauf des erfindungsgemäßen Verfahrens (a) durch das folgende Formelschema darstellen:

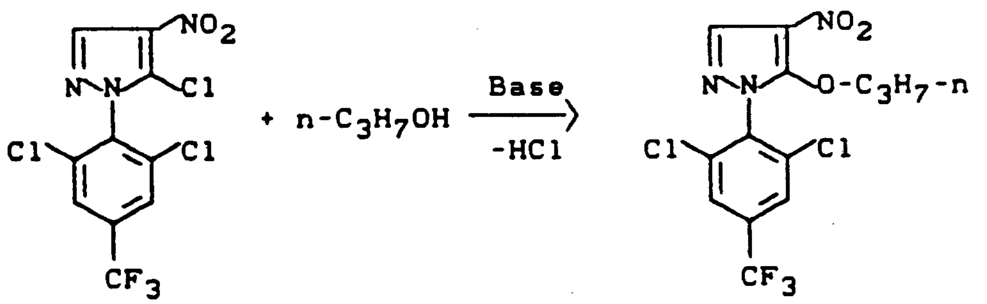


Verwendet man beispielsweise 1-(2-Chlor-4-trifluormethoxyphenyl)-3-methyl-5-methoxycarbonylmethoxy-pyrazol und Salpetersäure als Ausgangsstoffe, so läßt sich der Reaktionsablauf des erfindungsgemäßen Verfahrens (b) durch das folgende Formelschema darstellen:

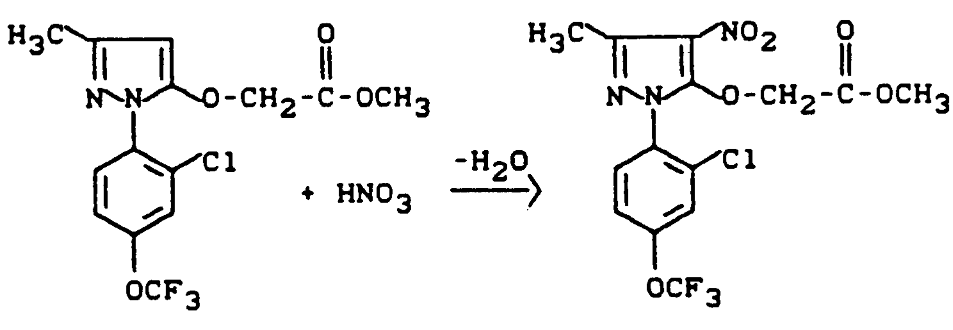


Verwendet man beispielsweise 4-Cyano-1-(2,4-dichlor-phenyl)-pyrazolin-5-thion und Methyliodid als Ausgansstoffe, so läßt sich der Reaktionsablauf des erfindungsgemäßen Verfahrens (c) durch das

folgende Formelschema darstellen:

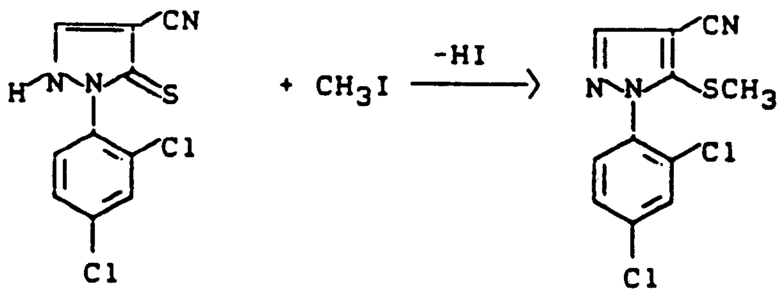


Verwendet man beispielsweise 1 – (2 – Brom – 4 – trifluormethylphenyl) – 5 – methylthio – 4 – nitro – pyrazol und Wasserstoffperoxid als Ausgangsstoffe, so läßt sich der Reaktionsablauf des erfindungsgrmäßen Verfahrens (d) durch das folgende Formelschema darstellen:

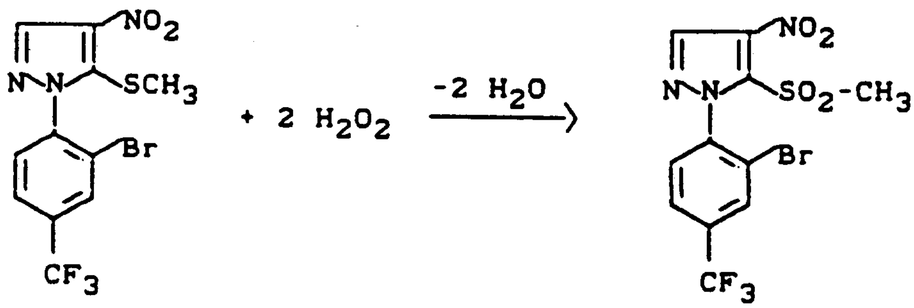


Verwendet man beispielsweise 4 – Cyan – 5 – methylsulfonyl – 1 – (2,4,6 – trichlor – phenyl) – pyrazol und Hydroxyessigsäuredimethylamid als Ausgangsstoffe, so läßt sich der Reaktionsablauf des erfindungsge – mäßen Verfahrens (e) durch das folgende Formelschema darstellen:

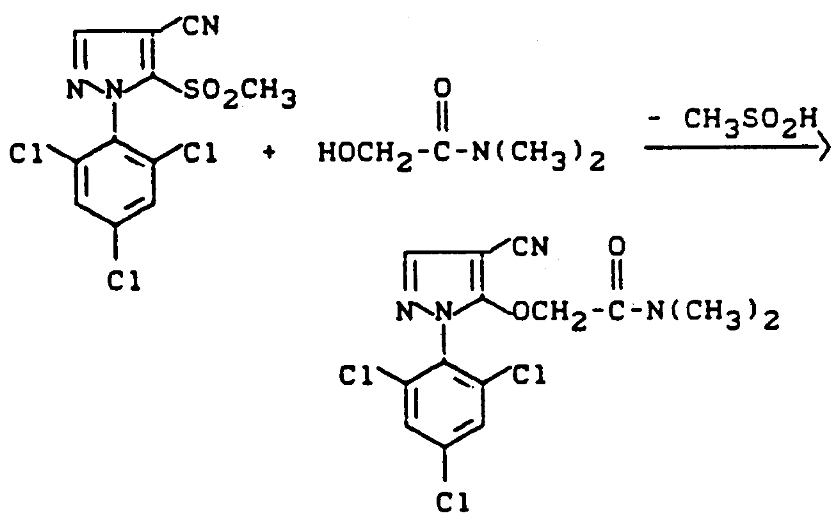


Die zur Durchführung des erfindungsgemäßen Verfahrens (a) als Ausgangsstoffe benötigten 5 – Halogen – pyrazol – Derivate sind durch die Formel (II) allgemein definiert. In dieser Formel stehen $R^1$, $R^2$ und Ar vorzugsweise für diejenigen Bedeutungen, die bereits im Zusammenhang mit der Beschreibung der erfindungsgemäßen Stoffe der Formel (I) als bevorzugt für diese Substituenten genannt wurden.

Die 5 – Halogen – pyrazol – Derivate der Formel (II) sind noch nicht bekannt; sie sind jedoch teilweise Gegenstand eigener, älterer Patentanmeldungen, die noch nicht veröffentlicht sind (vgl. die deutschen

Patentanmeldungen P 35 20 329 vom 31.05.1985, P 35 01 323 vom 15.01.1985 und P 35 28 476 vom 08.08.1985) und können nach den dort beschriebenen Verfahren erhalten werden, indem man beispielsweise
5-Amino-pyrazole der allgemeinen Formel (VIII)

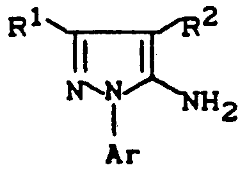

(VIII)

in welcher
$R^1$, $R^2$ und Ar die oben angegebene Bedeutung haben,
entweder mit Nitritverbindungen der Formel (IX)

$R^7-O-N=O$ (IX)

in welcher
$R^7$ für Wasserstoff, ein Alkalimetallkation oder Alkyl steht,
und mit einem Haloform der Formel (X)

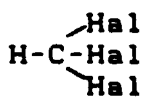

(X)

in welcher
Hal die oben angegebene Bedeutung hat,
oder mit Natriumnitrit in Gegenwart einer Halogenwasserstoffsäure der Formel (XI)

H - Hal (XI)

in welcher
Hal die oben angegebene Bedeutung hat,
jeweils gegebenenfalls in Gegenwart eines Verdünnungsmittels, wie beispielsweise überschüssiges Haloform bzw. überschüssige Halogenwasserstoffsäure und gegebenenfalls in Gegenwart eines Katalysators, wie beispielsweise eine Säure, bei Temperaturen zwischen -20 und +120°C umsetzt;
oder indem man Halogen-pyrazole der allgemeinen Formel (XII)

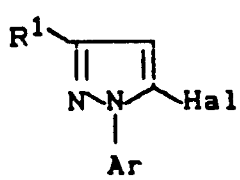

(XII)

in welcher
$R^1$, Ar und Hal die oben angegebene Bedeutung haben,
mit einem Nitrierungsmittel, wie beispielsweise Salpetersäure, entsprechend den Angaben zur Verfahrensvariante (b) umsetzt.

Die 5-Amino-pyrazole der Formel (VIII) sind bekannt (vgl. z.B. DE-OS 33 37 543 und DE-OS 34 02 308) bzw. können nach den dort beschriebenen Verfahren erhalten werden.

Die Nitritverbindungen der Formel (IX), die Haloforme der Formel (X) und die Halogenwasserstoffsäuren der Formel (XI) sind allgemein bekannte Verbindungen der organischen Chemie.

Die Halogen-pyrazole der Formel (XII) sind noch nicht bekannt. Sie können erhalten werdem, indem man Alkoxymethylenmalonester der Formel (XIII)

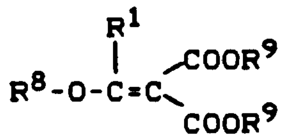

(XIII)

in welcher

$R^1$ die oben angegebene Bedeutung hat und

$R^8$ und $R^9$ unabhängig voneinander für Alkyl, vorzugsweise Methyl oder Ethyl, stehen,

mit Arylhydrazinen der Formel (XIV)

$Ar - NHNH_2$ (XIV)

in welcher

Ar die oben angegebene Bedeutung hat,

zunächst in einer ersten Stufe gegebenenfalls in Gegenwart eines Verdünnungsmittels, wie beispielsweise Methanol oder Ethanol, bei Temperaturen zwischen 10 ° C und 80 ° C umsetzt

– [Das hierbei intermediär auftretende Zwischenprodukt der Formel (XIVa)

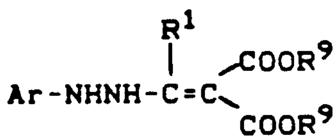

(XIVa)

in welcher

$R^1$, $R^9$ und Ar die oben angegebene Bedeutung haben,

kann gegebenenfalls auch isoliert und in einer separaten Reaktionsstufe cyclisiert werden] –
und die so erhältlichen Pyrazolcarbonsäureester der Formel (XV)

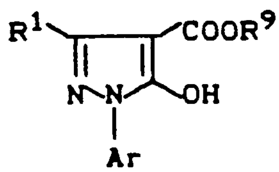

(XV)

in welcher

$R^1$, $R^9$ und Ar die oben angegebene Bedeutung haben,

in einer zweiten Stufe gegebenenfalls in Gegenwart eines Verdünnungsmittels, wie beispielsweise Methanol, und gegebenenfalls in Gegenwart einer Base, wie beispielsweise Natriumhydroxid, bei Temperaturen zwischen 30 ° C und 70 ° C decarboxyliert.

Die Cyclisierung und anschließende Decarboxylierung kann gegebenenfalls auch in einer Reaktionsstufe als 'Eintopfverfahren' durchgeführt werden (vgl. z.B. Liebigs Ann. Chem. 373, 142 (1910) sowie die Herstellungsbeispiele).

In einer dritten Stufe werden die so erhaltenen Pyrazolinone der Formel (XVIa)

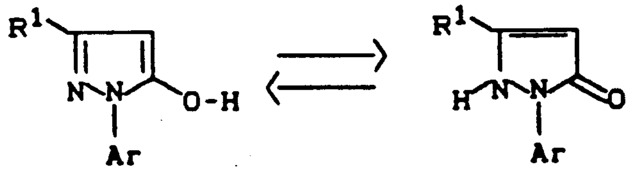

(XVIa)

in welcher

$R^1$ und Ar die oben angegebene Bedeutung haben,

mit Halogenierungsmitteln, wie beispielsweise Phosphoroxychlorid oder Phosphoroxybromid nach üblichen,

bekannten Verfahren umgesetzt (vgl. z.B. Ber. dtsch. Chem. Gesellschaft 28, 35 (1895) oder Liebigs Ann. Chem. 373, 129 (1910)).

Die Alkoxymethylenmalonester der Formel (XIII) sind allgemein bekannten Verbindungen der organischen Chemie.

Die Arylhydrazine der Formel (XIV) sind bekannt bzw. können nach bekannten Verfahren erhalten werden (vgl. z.B. Houben-Weyl, Methoden der organischern Chemie, Band X, 2; S. 203, Thieme Verlag Stuttgart 1967).

Die Pyrazolinone der Formel (XVI) können auch erhalten werden, indem man $\beta$-Ketoester der Formel (XVII)

$R^1-CO-CH_2-COOR^9$ (XVII)

in welcher

$R^1$ und $R^9$ die oben angegebene Bedeutung haben,

mit Arylhydrazinen der Formel (XIV) gegebenenfalls in Gegenwart eines Verdünnungsmittels, wie beispielsweise Toluol, und gegebenenfalls in Gegenwart eines Katalysators, wie beispielsweise p-Toluolsulfonsäure, bei Temperaturen zwischen 0°C und 120°C umsetzt (vgl. auch die Herstellungsbeispiele).

Die außerdem zur Durchführung des erfindunsggemäßen Verfahrens (a) als Ausgangsstoffe benötigten nucleophilen Verbindungen sind durch die Formel (III) allgemein definiert. In dieser Formel stehen $R^3$ und Y vorzugsweise für diejenigen Bedeutungen, die bereits im Zusammenhang mit der Beschreibung der erfindungsgemäßen Stoffe der Formel (I) als bevorzugt für diese Substituenten genannt wurden.

Die nucleophilen Verbindungen der Formel (III) sind allgemein bekannte Verbindungen der organischen Chemie.

Die zur Durchführung des erfindungsgemäßen Verfahrens (b) als Ausgangsstoffe benötigten 5-Oxy-(thio)-pyrazol-Derivate sind durch die Formel (IV) allgemein definiert. In dieser Formel stehen $R^1$, $R^3$ und Ar vorzugsweise für diejenigen Bedeutungen, die bereits im Zusammenhang mit der Beschreibung der erfindungsgemäßen Stoffe der Formel (I) als bevorzugt für diese Substituenten genannt wurden. $Y^1$ steht vorzugsweise für Sauerstoff oder Schwefel.

Die 5-Oxy(thio)-pyrazol-Derivate der Formel (IV) sind noch nicht bekannt. Sie können erhalten werden, indem man Pyrazolin-one(thione) der Formel (XVI)

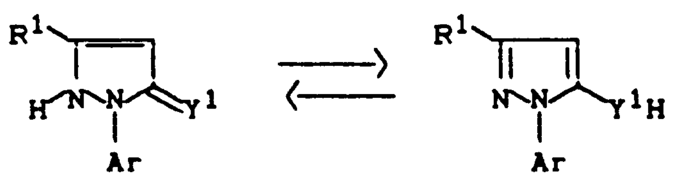

(XVI)

in welcher

$R^1$, Ar und $Y^1$ die oben angegebene Bedeutung haben,

mit Alkylierungsmitteln der Formel (VI) gemäß den Angaben des erfindungsgemäßen Verfahrens (c) umsetzt.

Die Pyrazolin-one der Formel (XVIa)

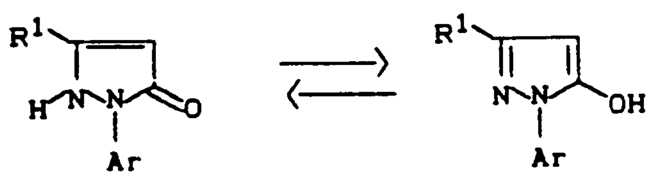

(XVIa)

in welcher

$R^1$ und Ar die oben angegebene Bedeutung haben,

können entsprechend den obigen Angaben des erfindungsgemäßen Verfahrens (a) erhalten werden.

Die Pyrazolin-thione der Formel (XVIb)

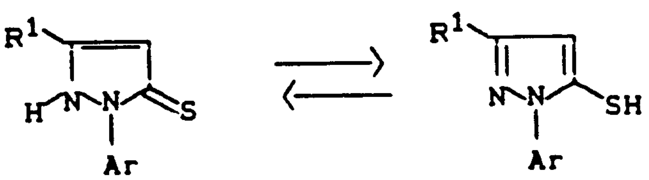

(XVIb)

in welcher

$R^1$ und Ar die oben angegebene Bedeutung haben,

können erhalten werden, indem man die Pyrazolin-one der Formel (XVIa) mit üblichen Schwefelreagenzien, wie beispielsweise Phosphorpentasulfid, in allgemein üblicher und bekannter Art und Weise umsetzt.

Die zur Durchführung des erfindungsgemäßen Verfahrens (c) als Ausgangsstoffe benötigten Pyrazolin-one(thione) sind durch die Formel (V) allgemein definiert. In dieser Formel stehen $R^1$, $R^2$ und Ar vorzugsweise für diejenigen Bedeutungen, die bereits im Zusammenhang mit der Beschreibung der erfindungsgemäßen Stoffe der Formel (I) als bevorzugt für diese Substituenten genannt wurden. $Y^1$ steht vorzugsweise für Sauerstoff oder Schwefel.

Die Pyrazolin-one(thione) der Formel (V) sind noch nicht bekannt. Sie können erhalten werden, indem man beispielsweise

Pyrazolin-one der Formel (XVIa) bzw. Pyrazolin-thione der Formel (XVIb) mit einem Nitrierungsmittel, wie beispielsweise Salpetersäure, gemäß den Angaben des erfindungsgemäßen Verfahrens (b) umsetzt; oder indem man

5-Halogen-pyrazol-Derivate der Formel (II) in allgemein üblicher Art und Weise mit Wasser oder mit wäßrigen Alkali- bzw. Erdalkalimetall-Basen hydrolysiert oder mit Schwefelwasserstoff bzw. mit Alkali- oder Erdalkalimetallsalzen des Schwefelwasserstoffs thiolysiert.

Die außerdem zur Durchführung des erfindungsgemäßen Verfahrens (c) als Ausgangsstoffe benötigten Alkylierungsmittel sind durch die Formel (VI) allgemein definiert. In dieser Formel steht $R^3$ vorzugsweise für diejenigen Bedeutungen, die bereits im Zusammenhang mit der Beschreibung der erfindungsgemäßen Stoffe der Formel (I) als bevorzugt für diesen Substituenten genannt wurden. A steht vorzugsweise für Chlor, Brom, Iod, p-Toluolsulfonyloxy oder Methoxysulfonyloxy.

Die Alkylierungsmittel der Formel (VI) sind allgemein bekannte Verbindungen der organischen Chemie.

Die zur Durchführung des erfindungsgemäßen Verfahrens (d) als Ausgangsstoffe benötigten 5-Thiopyrazol-Derivate der Formel (Id) sind erfindungsgemäße Verbindungen und entsprechend den erfindungsgemäßen Verfahren (a), (b) bzw. (c) erhältlich.

Die Oxidation gemäß dem erfindungsgemäßen Verfahren (d) erfolgt durch Umsetzung mit üblichen anorganischen und organischen Oxidationsmitteln.

Hierzu gehören vorzugsweise organische Persäuren, wie z.B. Peressigsäure, p-Nitroperbenzoesäure, m-Chlorperbenzoesäure; anorganische Persäuren, wie z.B. Periodsäure; weiterhin Wasserstoffperoxid in Eisessig oder Methanol, Kaliumpermanganat oder Chromsäure.

Die zur Durchführung des erfindungsgemäßen Verfahrens (e) als Ausgangsstoffe benötigten 5-Sulfonyl-pyrazol-Derivate der Formel (Ie) sind erfindungsgemäße Verbindungen und entsprechend den erfindungsgemäßen Verfahren (a) oder (d) erhältlich.

Die außerdem zur Durchführung des erfindungsgemäßen Verfahrens (e) als Ausgangsstoffe benötigten nucleophilen Verbindungen sind durch die Formel (IIIa) allgemein definiert. In dieser Formel steht $R^3$ vorzugsweise für diejenigen Bedeutungen, die bereits im Zusammenhang mit der Beschreibung der erfindungsgemäßen Stoffe der Formel (I) als bevorzugt für diesen Substituenten genannt wurden. $Y^1$ steht vorzugsweise für Sauerstoff oder Schwefel.

Die nucleophilen Verbindungen der Formel (IIIa) sind allgemein bekannte Verbindungen der organischen Chemie.

Als Verdünnungsmittel zur Durchführung der Herstellungsverfahren (a), (c) und (e) kommen inerte organische Lösungsmittel in Frage.

Hierzu gehören insbesondere aliphatische und aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Alkohole, wie Methanol, Ethanol oder Isopropanol, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid. In manchen Fällen erweist es sich als vorteilhaft, in wässrigorganischen homogenen oder Zweiphasen-systemen (Phasen-Transfer-Katalyse) zu arbeiten.
In einer besonderen Ausführungsform der Verfahren (a), (c) und (e) werden die Alkali- oder Erdalkali-Salze der nucleophilen Verbindungen der Formel (III), bzw. der Pyrazolin-one(thione) der Formel (V) als Reaktionspartner eingesetzt.

Die Herstellungsverfahren (a), (c) und (e) werden gegebenenfalls in Gegenwart eines Säurebindemittels durchgeführt.

Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, Alkalimetallhydride, wie Natriumhydrid, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei den erfindungsgemäßen Verfahren (a) und (e) in einem größeren Bereich variiert werden. Im allgemeinen arbeitet man bei Temperaturen zwischen -20 °C °C und +120 °C, vorzugsweise bei Temperaturen zwischen -10 °C und +80 °C.

Die Reaktionstemperaturen können bei dem erfindungsgemäßen Verfahren (c) in einem größeren Bereich variiert werden. Im allgemeinen arbeitet man bei Temperaturen zwischen -20 °C und +80 °C, vorzugsweise bei Temperaturen zwischen 0 °C und +50 °C.

Zur Durchführung des Herstellungsverfahrens (a) setzt man pro Mol 5-Halogen-pyrazol-Derivat der Formel (II) im allgemeinen 1 bis 10 Mol, vorzugsweise 1 bis 5 Mol an nucleophiler Verbindung der Formel (III) und gegebenenfalls 1 bis 5 Mol an Säurebindemittel ein. Die Reaktionsführung, Aufarbeitung und Isolierung der Reaktionsprodukte erfolgt nach allgemein bekannten und üblichen Verfahren.

Zur Durchführung des Herstellungsverfahrens (c) setzt man pro Mol Pyrazolin-one(thione) der Formel (V) im allgemeinen 1 bis 10 Mol, vorzugsweise 1 bis 5 Mol an Alkylierungsmittel der Formel (VI) und 1 bis 5 Mol Säurebindemittel ein. Die Reaktionsführung, Aufarbeitung und Isolierung der Reaktionsprodukte erfolgt nach allgemein bekannten und üblichen Verfahren.

Zur Durchführung des Herstellungsverfahrens (e) setzt man pro Mol 5-Sulfonyl-pyrazol-Derivat der Formel (Ie) im allgemeinen 1 bis 10 Mol, vorzugsweise 1 bis 5 Mol an nucleophiler Verbindung der Formel (IIIa) und gegebenenfalls 1 bis 5 Mol an Säurebindemittel ein. Die Reaktionsführung, Aufarbeitung und Isolierung der Reaktionsprodukte erfolgt nach allgemein bekannten und üblichen Verfahren.

Als Nitrierungsmittel zur Durchführung des Herstellungsverfahrene (b) kommen vorzugsweise Salpetersäure und Nitriersäure infrage.

Als Verdünnungsmittel zur Durchführung des erfindungsgemäßen Verfahrens (b) kommen alle üblicherweise für derartige Nitrierungsreaktionen verwendbaren Lösungsmittel infrage. Vorzugsweise verwendet man die als Reagenzien infrage kommenden Säuren oder deren Gemische mit Katalysatorsäuren, wie beispielsweise Schwefelsäure, Salpetersäure, Acetanhydrid oder Nitriersäure, gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff als Verdünnungsmittel infrage.

Als Katalysatoren zur Durchführung des erfindungsgemäßen Verfahrens (b) kommen ebenfalls die für derartige Nitrierungen üblichen Katalysatoren infrage; vorzugsweise verwendet man saure Katalysatoren, wie beispielsweise Schwefelsäure oder Acetanhydrid.

Die Reaktionstemperaturen können bei der Durchführung des erfindungsgemäßen Verfahrens (b) in einem größeren Bereich variiert werden. Im allgemeinen arbeitet man zwischen -50 °C und +200 °C, vorzugsweise zwischen -20 °C und +150 °C.

Zur Durchführung des erfindungsgemäßen Verfahrens (b) setzt man pro Mol 5-Oxy(thio)pyrazol-Derivat der Formel (IV) im allgemeinen 1,0 bis 100 Mol, vorzugsweise 1,0 bis 50 Mol an Salpetersäure und gegebenenfalls 0,1 bis 10 Mol an Katalysator ein.

Die Reaktionsführung, Aufarbeitung und Isolierung der Reaktionsprodukte erfolgt in allgemein üblicher Art und Weise.

Die Reaktionstemperaturen können bei der Durchführung der Oxidation gemäß Herstellungsverfahren (d) in einem größeren Bereich variiert werden. Im allgemeinen arbeitet man zwischen -50 °C und 100 °C, vorzugsweise zwischen -30 °C und 80 °C.

Bei der Durchführung der erfindungsgemäßen Oxidation gemäß Herstellungsverfahren (d) setzt man auf 1 Mol 5-Thio-pyrazol-Derivate der Formel (I d) etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Acetanhydrid bei Temperaturen zwischen -30 °C bis +30 °C, entstehen vorzugsweise die

erfindungsgemäßen Verbindungen der Formel (Ic) mit SO-Gruppierung. Bei Überschuß an Oxidationsmittel und höheren Temperaturen (10 bis 80°C) entstehen vorzugsweise die erfindungsgemäßen Verbindungen der Formel (Ic) mit $SO_2$-Gruppierung. Die Isolierung der Oxidationsprodukte erfolgt in üblicher Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernie, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von mono- und dikotylen Unkräutern in mono- und dikotylen Kulturen, wie beispielsweise Baumwolle, Weizen, Mais und Gerste, im Vor- und im Nachauflaufverfahren einsetzen.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Suspensions-Emulsions-Konzentrete, wirkstoffimprägnierte Natur- und synthetische Stoffe, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie
Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeeerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mengen, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Bei der Anwendung als Herbizide können die erfindungsgemäßen Wirkstoffe als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5-(4H)-on zur Unkrautbekämpfung in Sojabohnen, in Frage. Auch Mischung mit N,N-Dimethyl-N'-(3-trifluormethylphenyl)-harnstoff; N,N-Dimethyl-N'-(3-chlor-4-methylphenyl)-harnstoff; N,N-Dimethyl-N'-(4-isopropylphenyl)-harnstoff; 4-Amino-6-t-butyl-3-ethylthio-1,2,4-triazin-5-(4H)-on; 2,4-Dichlorphenoxyessigsäure; (2-Methyl-4-chlorphenoxy)-essigsäure; (4-Chlor-2-methylphenoxy)-propionsäure; Chloressigsäure-N-(methoxymethyl)-2,6-diethylanilid; 2-Ethyl-6-methyl-N-(1-methyl-2-methoxyethyl)-chloracetanilid; 2-[4-(3,5-Dichlorpyrid-2-yloxy)-phenoxy]-propionsäure-(trimethylsilylme thylester); Methyl-5-(2,4-dichlorphenoxy)-2-nitrobenzoat; 3,5-Diiod-4-hydroxybenzonitril; 3-Isopropyl-2,1,3-benzothiadiazin-4-on-2,2-dioxid; 2-Chlor-N-{[(4-methoxy-6-methyl-1,3,5-triazin-2-yl)-amino]-carbonyl}-benzolsulfonamid; 4-Ethylamino-2-t-butylamino-6-methylthio-s-triazin; N-Methyl-2-(1,3-benzthiazol-2-yloxy)-acetanilid; N,N-Di-n-propyl-thiocarbamidsäure-S-ethylester; exo-1-Methyl-4-(1-methylethyl)-2-(2-methylphenyl-methoxy)-7-oxabicyclo-(2,2,1)-heptan; 2-{⟨3-Chlor-5-(trifluormethyl)-2-pyridinyl⟩-oxy]-phenoxy}-propansäure bzw. -propansäureethylester; N,N-Diisopropyl-S-(2,3,3-trichlorallyl)-thiolcarbamat; 2-Chlor-4-ethylamino-6-isopropylamino-1,3,5-triazin; 2-Chlor-4-ethylamino-6-(3-cyanopropylamino)-1,3,5-triazin; [(4-Amino-3,5-dichlor-6-fluor-2-pyridinyl)-oxy]-essigsäure; 3,5-Dibrom-4-hydroxy-benzonitril; 2-[4-(2,4-Dichlorphenoxy)-phenoxy]-propionsäure-methylester ist möglich.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Bei Anwendung im Nachauflaufverfahren können die erfindungsgemäßen Verbindungen alleine oder in Kombination mit emulgierbaren Ölen, oberflächenaktiven Substanzen und anderen Additiven ausgebracht werden.

Sie können auch vor der Saat in den Boden eingearbeitet werden.

Die angewandte Wirkstoffmenge kann in einem größeren Bereich schwanken, Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Bei der Anwendung als Wachstumsregulatoren können die erfindungsgemäßen Wirkstoffe in den Formulierungen ebenfalls in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Die Aufwandmengen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungsseit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele

Beispiel 1

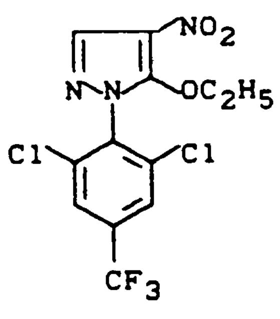

(I-1)

(Verfahren a)

11 g (0,03 Mol) 5-Chlor-1-(2,6-dichlor-4-trifluormethylphenyl)-4-nitro-pyrazol in 30 ml Tetrahydrofuran wird bei 25 °C tropfenweise zu einer Mischung aus 1 g metallischem Natrium in 30 ml Ethanol und 30 ml Tetrahydrofuran gegeben. Es wird noch 3 Stunden bei 25 °C gerührt, die Reaktionsmischung sodann in Wasser eingegossen, die wässrige Phase mit Toluol extrahiert, über Natriumsulfat getrocknet und im Vakuum eingeengt.

Man erhält 4,1 g (37 % der Theorie) 1-(2,6-Dichlor-4-trifluormethyl-phenyl)-5-ethoxy-4-nitro-pyrazol vom Schmelzpunkt 84-86 °C.

Herstellung des Ausgangsproduktes

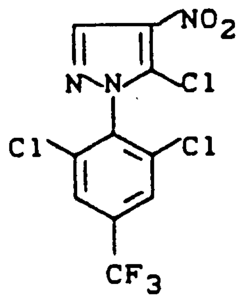

(II-1)

62 g (0,2 Mol) 5-Chlor-1-(2,6-dichlor-4-trifluormethylphenyl)-pyrazol werden in einer Mischung aus 260 ml konzentrierter Schwefelsäure und 70 ml Wasser suspendiert und bei 60 °C tropfenweise mit einem Gemisch aus 40 ml konzentrierter Schwefelsäure und 40 ml konzentrierter Salpetersäure versetzt. Nach beendeter Zugabe wird noch 12 Stunden bei 60 °C gerührt, abgekühlt und auf Eis gegossen. Das sich ölig abscheidende Reaktionsprodukt wird mit Toluol aufgenommen, die organische Phase mehrmals mit Wasser und dann mit Natriumhydrogencarbonat-Lösung gewaschen und anschließend über Natriumsulfat getrocknet. Nach Abdestillieren des Lösungsmittels im Vakuum wird der Rückstand aus wenig Petrolether umkristallisiert.

Man erhält 32 g (44 % der Theorie) an 5-Chlor-1-(2,6-dichlor-4-trifluormethylphenyl)-4-nitro-pyrazol vom Schmelzpunkt 80 °C-84 °C.

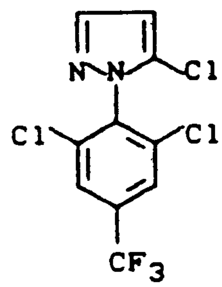

(XII-1)

Ein Gemisch aus 60 g (0,2 Mol) 1-(2,6-Dichlor-4-trifluormethyl-phenyl)-5-hydroxy-pyrazol und 250 ml Phosphoroxychlorid werden im Autoklaven 20 Stunden auf 160 °C erhitzt. Anschließend wird der abgekühlte Reaktionsansatz auf Eis gegossen und das überschüssige Phosphoroxychlorid vorsichtig unter Kühlen mit Natronlauge hydrolysiert. Der Niederschlag wird abgesaugt, mehrmals mit Wasser gewaschen und getrocknet.

Man erhält 32 g (51 % der Theorie) an 5-Chlor-1-(2,6-dichlor-4-trifluormethyl-phenyl)-pyrazol vom Schmelzpunkt 70 °C-72 °C.

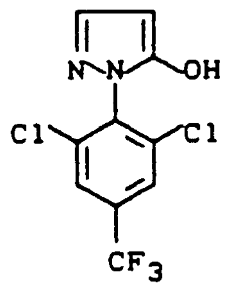

(XVIa-1)

In eine Lösung aus 30 g (0,75 Mol) Natriumhydroxid in 1000 ml Wasser trägt man bei 80-85 °C portionsweise unter Rühren 105 g (0,253 Mol) fein gepulverten β-(2,6-Dichlor-4-trifluormethyl-phenyl)-hydrazinomethylenmalonsäurediethylester und rührt anschließend weitere 48 Stunden bei 97-98 °C.

Die erkaltete Reaktionsmischung wird mit konzentrierter Salzsäure vorsichtig angesäuert auf pH 2 und der so erhaltene Niederschlag abgesaugt und auf Ton getrocknet.

Man erhält 100 g (67 % der Theorie) an 5 – Hydroxy – 1 – (2,6 – dichlor – 4 – trifluormethyl – phenyl) – pyrazol vom Schmelzpunkt 223 ° C – 225 ° C.

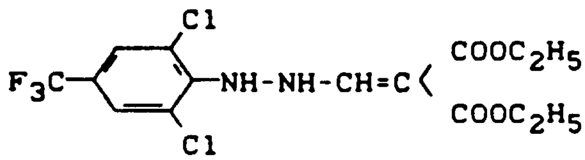

(XIVa-1)

Zu einer Lösung von 122,5 g (0,5 Mol) 2,6 – Dichlor – 4 – trifluormethyl – phenylhydrazin in 1000 ml Ethanol tropft man bei 70 ° C – 75 ° C innerhalb von 30 Minuten unter Rühren 115 g (0,53 Mol) Ethoxymethylenmalonsäurediethylester und rührt nach beendeter Zugabe weitere 5 Stunden bei 70 ° C bis 75 ° C. Zur Aufarbeitung entfernt man das Lösungsmittel im Vakuum, reibt den Rückstand mit Wasser an, saugt ab und trocknet auf Ton.

Man erhält 202 g (97 % der Theorie) an $\beta$ – (2,6 – Dichlor – 4 – trifluormethylphenyl) – hydrazinomethylen – malonsäurediethylester vom Schmelzpunkt 73 ° C – 83 ° C.

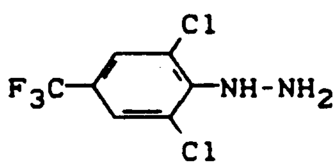

(XIV-1)

6,2 g (0,025 Mol) 3,4,5 – Trichlor – trifluormethylbenzol und 6,25 g (0,125 Mol) Hydrazinhydrat werden in 12 ml Pyridin 48 Stunden bei 115 – 120 ° C unter Rückfluß erhitzt. Zur Aufarbeitung destilliert man das Lösungsmittel ab, nimmt den Rückstand in Wasser auf und extrahiert dreimal mit jeweils ca. 30 ml Dichlormethan. Die vereinigten organischen Phasen werden über Magnesiumsulfat getrocknet, im Vakuum eingeengt und anschließend destilliert.

Man erhält 5,1 g (83 % der Theorie) an 2,6 – Dichlor – 4 – trifluormethylphenylhydrazin vom Schmelzpunkt 56 bis 57 ° C.

Beispiel 2

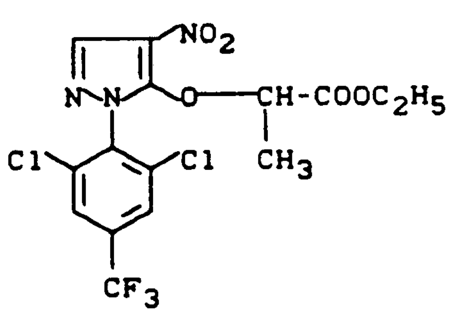

(I-2)

(Verfahren b)

17 g (0,05 Mol) 1 – (2,6 – Dichlor – 4 – trifluormethyl – phenyl) – 5 – (1 – ethoxycarbonyl – ethoxy) – pyrazol werden in 50 ml Acetanhydrid gelöst und unter Rühren bei 0 – 10 ° C innerhalb von 15 Minuten mit 10 ml konzentrierter Salpetersäure tropfenweise versetzt. Es wird noch 1 Stunde bei Raumtemperatur nachgerührt und der Reaktionsansatz dann auf Eiswasser gegossen. Nach Neutralisation der Mischung mit Natriumhydrogencarbonat werden die organischen Anteile mit Methylenchlorid extrahiert. Die Methylenchloridphase wird über Natriumsulfat getrocknet und im Vakuum eingeengt. Die Rohausbeute an öligem Reaktionsprodukt beträgt 17 g. Nach Verreiben mit Petrolether erhält man 10,8 g (49 % der Theorie) an 1 – (2,6 –

Dichlor-4-trifluormethyl-phenyl)-5-(1-ethoxycarbonyl-ethoxy)-4-nitro-pyrazol in Form von schmierigen Kristallen.

Herstellung des Ausgangsproduktes

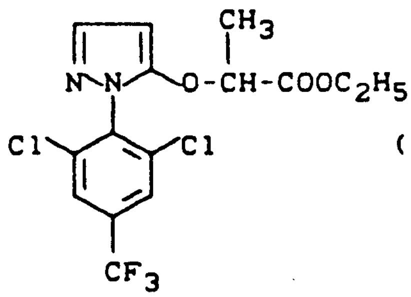

(IV-1)

Zu einer Mischung aus 9 g (0,03 Mol) 1-(2,6-Dichlor-4-trifluormethyl-phenyl)-4-H-$\Delta^2$-pyrazolin-on, 5,0 g (0,036 Mol) Kaliumcarbonat in 200 ml Acetonitril gibt man 5,4 g (0,03 Mol) α-Brompropionsäureethylester und rührt den Ansatz 12 Stunden bei 70 °C. Anschließend wird auf Wasser gegossen, die organischen Anteile mit Mehylenchlorid extrahiert, über Natriumsulfat getrocknet und im Vakuum eingeengt. Nach Destillation des Rückstandes gehen bei 135 °C/0,1 Torr 6 g (50 % der Theorie) 1-(2,6-Dichlor-4-trifluormethyl-phenyl)-5-(1-ethoxycarbonyl-ethoxy)-pyrazol als Öl mit einem Brechungsindex $n_D^{23}$ = 1,5043 über.

Beispiel 3

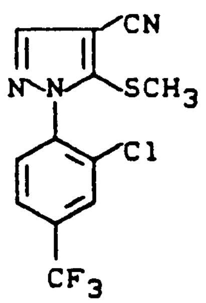

(I-3)

(Verfahren a)

Zu einer Suspension von 0,011 Mol Kalium-methylmercaptid, hergestellt durch Einleiten von 0,6 g (0,011 Mol) Methylmercaptan in eine Lösung von 1,4 g (0,012 Mol) Kalium-t-butylat in 50 ml Tetrahydrofuran, wird bei 20 - 25 °C eine Lösung von 3,5 g (0,01 Mol) 5-Brom-1-(2-chlor-4-trifluormethyl-phenyl)-4-cyano-pyrazol getropft. Es wird über Nacht gerührt, auf Wasser gegossen und 3 mal mit je 100 ml Methylenchlorid ausgeschüttelt. Nach Trocknen der vereinigten Phasen über Natriumsulfat wird die Lösung im Vakuum eingeengt. Es verbleibt ein schmieriger Rückstand, der nach Stehen über Nacht durchkristallisiert.
Man erhält 3,1 g (98 % der Theorie) 1-(2-Chlor-4-trifluormethyl-phenyl)-4-cyano-5-methylthio-pyrazol vom Schmelzpunkt 59-62 °C.

Herstellung des Ausgangsproduktes

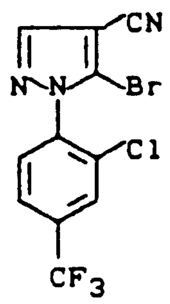

(II-2)

8,6 g (0,03 Mol) 5-Amino-1-(2-chlor-4-trifluormethylphenyl)-4-cyano-pyrazol werden in 100 ml 48 %ige Bromwasserstoffsäure suspendiert und bei -5°C bis 0°C unter Rühren tropfenweise mit einer Lösung von 3,6 g Natriumnitrit in 9 ml Wasser versetzt. Nach Beendigung der Stickstoff-Entwicklung wird noch 6 Stunden bei Raumtemperatur gerührt, der Niederschlag abgesaugt, mit einer wässrigen Natriumhydrogencarbonatlösung verrührt, nochmals abgesaugt und getrocknet.
Man erhält 10 g (95 % der Theorie) 5-Brom-1-(2-chlor-4-trifluormethylphenyl)-4-cyano-pyrazol vom Schmelzpunkt 127°C.

Beispiel 4

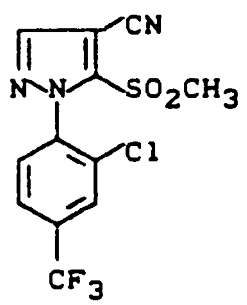

(I-4)

(Verfahren d)

3,2 g (0,01 Mol) 1-(2-Chlor-4-trifluormethyl-phenyl)-4-cyano-5-methylthio-pyrazol (Bsp. I-3) werden in 50 ml Methylenchlorid gelöst und bei 25°C mit einer Lösung von 3,5 g (0,02 Mol) 3-Chlorperbenzoesäure in 30 ml Methylenchlorid versetzt. Es wird über Nacht gerührt (ca. 12 h), dann noch 2 mal mit jeweils 1,8 g Persäure versetzt und insgesamt 17 Stunden gerührt, bis dünnschichtchromatografisch kein Ausgangsmaterial mehr nachzuweisen ist. Die Reaktionsmischung wird filtriert, und die Lösung mit gesättigter Natriumthiosulfat-Lösung geschüttelt bis keine Persäure mehr nachweisbar ist. Es wird nochmals filtriert, die Lösung dann mit gesättigter Natriumhydrogencarbonat-Lösung gewaschen, die organische Phase über Natriumsulfat getrocknet und im Vakuum eingeengt. Der kristalline Rückstand wird in heißem Toluol gelöst. Der Rückstand wird aus Toluol/Petrolether umkristallisiert. Es resultieren 2,6 g (75 % der Theorie) 1-(2-Chlor-4-trifluormethyl-phenyl)-4-cyano-5-methansulfonyl)-pyrazol vom Schmelzpunkt 126-133°C.

Beispiel 5

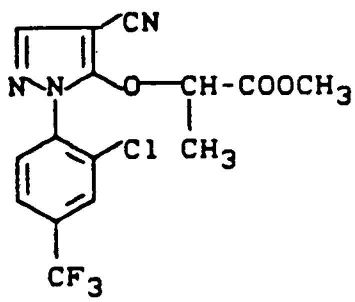

(I-5)

(Verfahren e)

Zu einer Suspension von 0,4 g (0,11 Mol) Natriumhydrid in 100 ml wasserfreiem Tetrahydrofuran werden tropfenweise 1,1 g (0,011Mol) Milchsäuremethylester gegeben. Nach Beendigung der Wasserstoff-entwicklung wird eine Lösung von 3,5 g (0,01 Mol) 1-(2-Chlor-4-trifluormethyl-phenyl)-4-cyano-5-methansulfonyl-pyrazol (Bsp.I-4) in Tetrahydrofuran zugetropft. Nach einstündigem Rühren bei Zimmertemperatur ist dünnschichtchromatographisch immer noch etwas vom Ausgangspyrazol nachzuweisen. Nach Zugabe von weiteren 0,04 g Natriumhydrid und 0,1 g Milchsäuremethylester und 2-stündigem Rühren ist dünnschichtchromatographisch kein Ausgangspyrazol mehr nachweisbar. Der Reaktionsansatz wird im Vakuum eingeengt, der Rückstand zwischen Wasser und Methylenchlorid verteilt, die Methylenchlorid-Phase mit Wasser gewaschen, über Natriumsulfat getrocknet und im Vakuum eingeengt. Der ölige Rückstand kristallisiert über Nacht durch. Nach Verreiben mit Petrolether und Absaugen erhält man 2,3 g (62 % der Theorie) 1-(2-Chlor-4-trifluormethyl-phenyl)-4-cyano-5-(1-methoxycarbonyl-ethoxy)-pyrazol vom Schmelzpunkt 82-85 °C.

Entsprechend den Herstellungsbeispielen und gemäß den allgemeinen Angaben zu den Verfahren (a) bis (e) erhält man die folgenden Endprodukte der allgemeinen Formel (I)

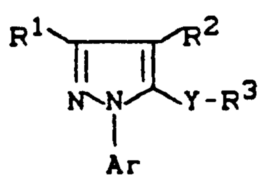

(I)

Tabelle 2

| Bsp. Nr. | $R^1$ | $R^2$ | $R^3$ | Y | Ar | Fp (°C) bzw. $^1HNMR$ in $CDCl_3$ in ppm |
|---|---|---|---|---|---|---|
| I-6 | H | $NO_2$ | $CH_3$ | O | 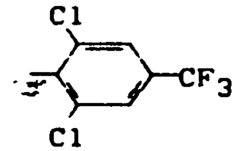 | 81-84 |
| I-7 | $CH_3$ | $NO_2$ | $CH_3$ | O | 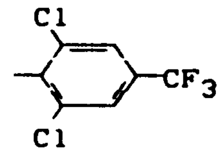 | 2,34(s,3H); 2,58(s,3H); 7,71(s,2H) |
| I-8 | $CH_3$ | $NO_2$ | $i-C_3H_7$ | O | 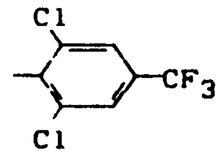 | 1,26(d,6H); 2,58(s,3H); 7,71(s,2H) |
| I-9 | H | $NO_2$ | 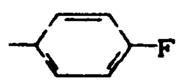 | O | 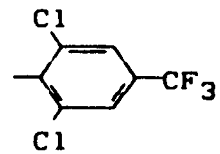 | 6,89(s,2H); 7,0(s,2H); 7,70(s,2H); 8,33(s,1H) |
| I-10 | $CH_3$ | $NO_2$ | $CH_3$ | S | 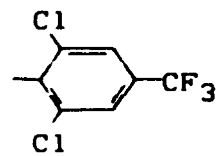 | 2,5 (s,3H); 2,64(s,3H); 7,74(s,2H) |
| I-11 | H | $NO_2$ | $CH_3$ | S | 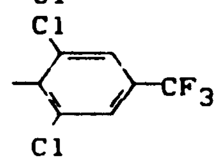 | 68-72 |

Tabelle 2 (Fortsetzung)

| Bsp. Nr. | $R^1$ | $R^2$ | $R^3$ | Y | Ar | Fp (°C) bzw. $^1$HNMR in $CDCl_3$ in ppm |
|---|---|---|---|---|---|---|
| I-12 | H | $NO_2$ | $-CH_2-COOCH_3$ | S | 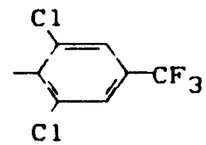 | 158-61 |
| I-13 | H | $NO_2$ | $CH_3$ | $SO_2$ | 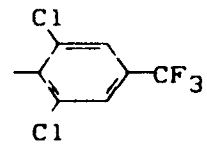 | 125-30 |
| I-14 | H | $NO_2$ | $CH_3$ | S | 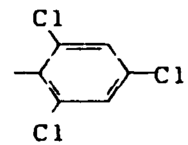 | 98 |
| I-15 | H | $NO_2$ | $CH_3$ | $SO_2$ | 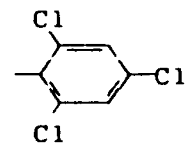 | 158 |
| I-16 | H | $NO_2$ | $CH_3$ | O | 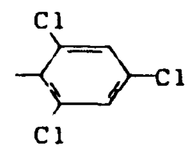 | 8,2(s,1H) 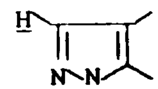 4,2(s,3H) O-C<u>H</u>$_3$ |
| I-17 | H | $NO_2$ | $C_3H_7$ | O | 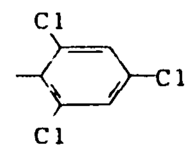 | 50-52 |

Tabelle 2 (Fortsetzung)

| Bsp. Nr. | $R^1$ | $R^2$ | $R^3$ | Y | Ar | Fp (°C) bzw. $^1HNMR$ in $CDCl_3$ in ppm |
|---|---|---|---|---|---|---|
| I-18 | H | $NO_2$ | $C_2H_5$ | O | 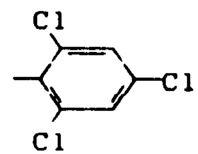 | 84 |
| I-19 | H | $NO_2$ | $CH_3$<br>\|<br>$-CH-COOC_2H_5$ | O | 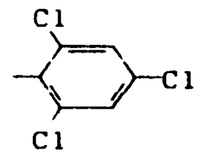 | 89 |
| I-20 | H | $NO_2$ | $C_2H_5$ | O | 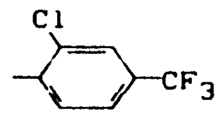 | 96 |
| I-21 | H | $NO_2$ | $C_3H_7$ | O | 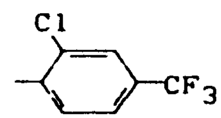 | 8,2(s,1H)<br>4,4(t,2H) 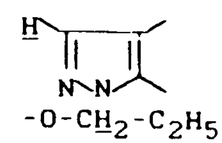 |
| I-22 | H | $NO_2$ | $CH_3$<br>\|<br>$-CH-COOC_2H_5$ | O | 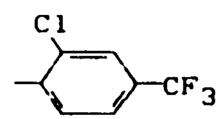 | 8,2(s,1H)<br>5,6(q,1H) 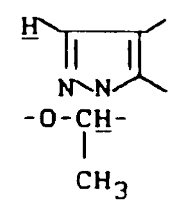 |

Tabelle 2 (Fortsetzung)

| Bsp. Nr. | $R^1$ | $R^2$ | $R^3$ | Y | Ar | Fp (°C) bzw. $^1HNMR$ in $CDCl_3$ in ppm |
|---|---|---|---|---|---|---|
| I-23 | H | CN | 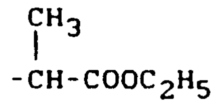 | O | 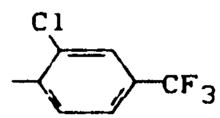 | 7,75(s,1H) 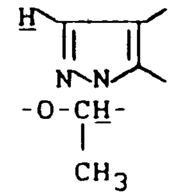 5,4(q,1H) |
| I-24 | H | $NO_2$ | 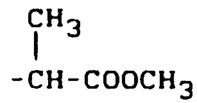 | O | 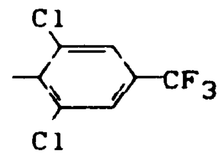 | 98 |
| I-25 | H | $NO_2$ | 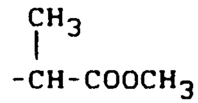 | O | 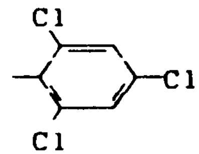 | 84-85 |
| I-26 | H | $NO_2$ | 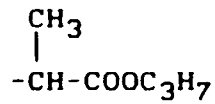 | O | 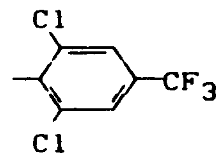 | 8,2(s,1H) 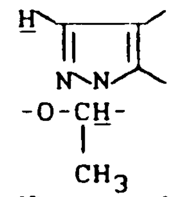 5,5(q,1H) |
| I-27 | H | $NO_2$ | 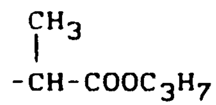 | O | 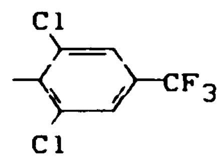 | 8,2(s,1H) 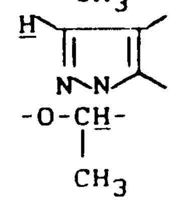 5,45(q,1H) |

Tabelle 2 Fortsetzung)

| Bsp. Nr. | $R^1$ | $R^2$ | $R^3$ | Y | Ar | Fp (°C) bzw. $^1HNMR$ in $CDCl_3$ in ppm |
|---|---|---|---|---|---|---|
| I-28 | H | CN | $CH_3$ | $SO_2$ | 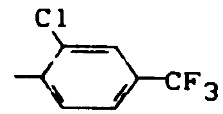 | 85-90 |
| I-29 | H | CN | $-CH_2-$ 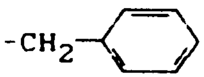 | S | 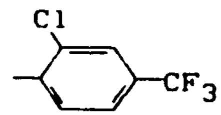 | 8,0(s,1H) 4,1(s,2H) 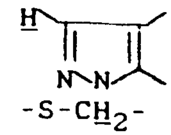 |
| I-30 | H | CN | $-CH_2-$ 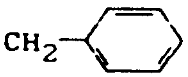 | S | 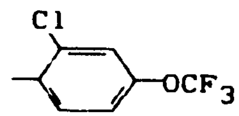 | 8,1(s,1H) 4,1(q,1H) 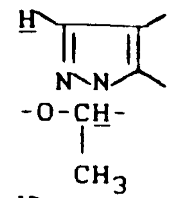 |
| I-31 | H | $NO_2$ | 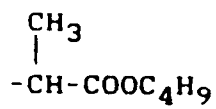 | O | 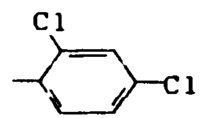 | 8,25(s,1H) 5,6(q,1H) 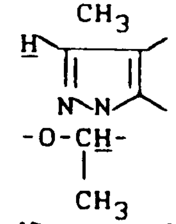 |
| I-32 | H | $NO_2$ | 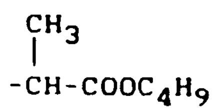 | O | 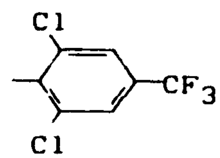 | 8,25(s,1H) 5,5(q,1H) 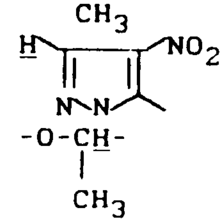 |

Tabelle 2 (Fortsetzung)

| Bsp. Nr. | $R^1$ | $R^2$ | $R^3$ | Y | Ar | Fp (°C) bzw. $^1HNMR$ in $CDCl_3$ in ppm |
|---|---|---|---|---|---|---|
| I-33 | H | CN | 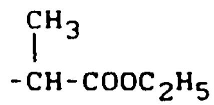 | O | 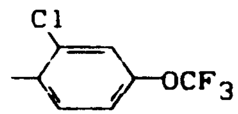 | 7,75(s,1H) 5,4(q,1H) 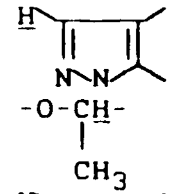 |
| I-34 | H | $NO_2$ | 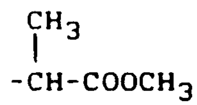 | O | 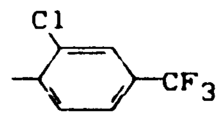 | 8,25(s,1H) 5,6(q,1H) 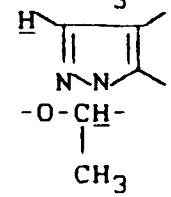 |
| I-35 | H | CN | $CH_3$ | O | 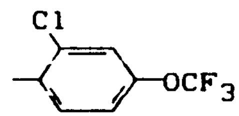 | 70 |
| I-36 | H | CN | $C_2H_5$ | O | 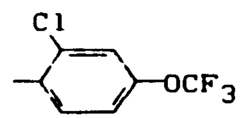 | 7,75(s,1H) 4,65 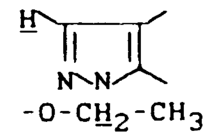 |
| I-37 | H | CN | $CH_3$ | O | 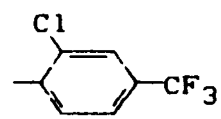 | 104 |

Tabelle 2 (Fortsetzung)

| Bsp. Nr. | $R^1$ | $R^2$ | $R^3$ | Y | Ar | Fp (°C) bzw. $^1$HNMR in $CDCl_3$ in ppm |
|---|---|---|---|---|---|---|
| I-38 | H | CN | $CH_3$<br>\|<br>$-CH-COOCH_3$ | O | 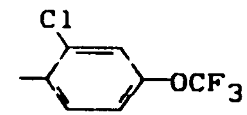 | 74-78 |
| I-39 | H | CN | $CH_3$<br>\|<br>$-CH-COOC_4H_9$ | O | 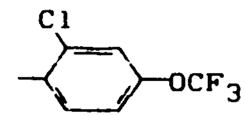 | 7,75(s,1H)<br>5,45(q,1H) 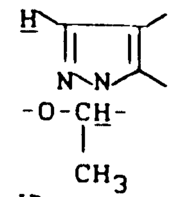 |
| I-40 | H | CN | $-CH_2-COOC_4H_9$ | O | 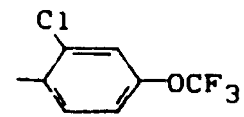 | 7,8(s,1H)<br>5,0(s,2H) 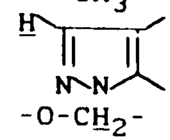 |
| I-41 | H | $NO_2$ | $-CH_2-COOC_4H_9$ | O | 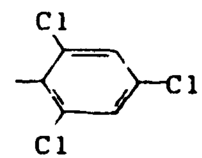 | 8,3(s,1H)<br>5,05(s,2H) 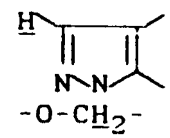 |
| I-42 | H | $NO_2$ | $CH_3$<br>\|<br>$-CH-COOC_2H_5$ | O | 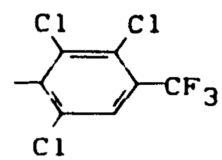 | 8,25(s,1H)<br>5,45(m,1H) 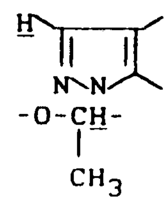 |

Tabelle 2 (Fortsetzung)

| Bsp. Nr. | $R^1$ | $R^2$ | $R^3$ | Y | Ar | Fp (°C) bzw. $^1$HNMR in $CDCl_3$ in ppm |
|---|---|---|---|---|---|---|
| I-43 | H | $NO_2$ | $CH_3$<br>\|<br>$-CH-COOC_3H_7$ | O | 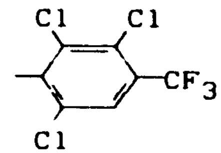 | 8,25(s,1H)<br>5,5(m,1H) 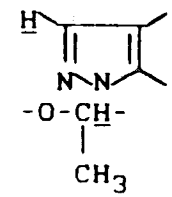 |
| 1-44 | H | $NO_2$ | $CH_3$ | S | 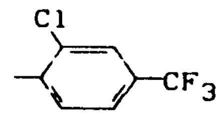 | 8,45(s,1H)<br>2,5(s,3H) 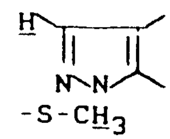 |
| I-45 | H | $NO_2$ | $CH_3$<br>\|<br>$-CH-COOCH_3$ | O | 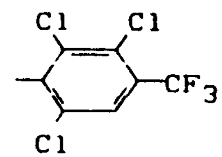 | 83 |

Tabelle 2 (Fortsetzung)

| Bsp. Nr. | $R^1$ | $R^2$ | $R^3$ | Y | Ar | physikalische Eigenschaften |
|---|---|---|---|---|---|---|
| I-46 | H | $NO_2$ | $-CH(CH_3)-COO(CH_2)_2CH_3$ | O | 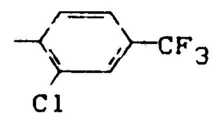 | $^1H$-NMR*): 8.2; 5.6 |
| I-47 | H | CN | $-CH_2-$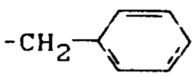 | SO | 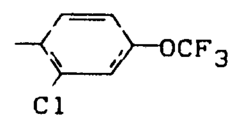 | Fp. 109°-116° C |
| I-48 | H | CN | $CH_3$ | S | 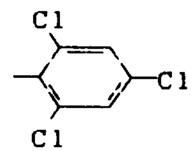 | Fp. 84°-91° C |
| I-49 | H | CN | $CH_3$ | $SO_2$ | 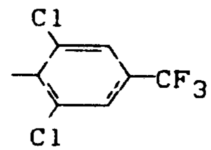 | Fp. 154°-156° C |
| I-50 | H | $NO_2$ | $-(CH_2)_3-CH_3$ | O | 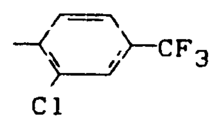 | $^1H$-NMR*): 8.1; 4.4 |

Tabelle 2 (Fortsetzung)

| Bsp. Nr. | $R^1$ | $R^2$ | $R^3$ | Y | Ar | physikalische Eigenschaften |
|---|---|---|---|---|---|---|
| I-51 | H | CN | 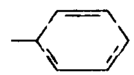 | O | 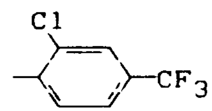 | Fp. 118° -120° C |
| I-52 | H | CN | 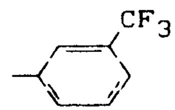 | O | 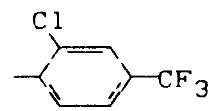 | Fp. 117° -121° C |
| I-53 | H | CN | $C_2H_5$ | O | 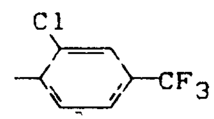 | Fp. 100° C |
| I-54 | H | CN | $CH_3$<br>\|<br>$-CH-COO-(CH_2)_3-CH_3$ | O | 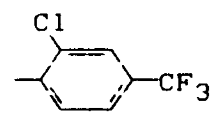 | $^1H$-NMR*): 7.8; 5.4 |
| I-55 | H | CN | $CH_3$<br>\|<br>$-CH-CO-NH-(CH_2)_2-CH_3$ | O | 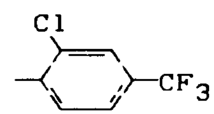 | Fp. 78° -87° C |

Tabelle 2 (Fortsetzung)

| Bsp. Nr. | $R^1$ | $R^2$ | $R^3$ | Y | Ar | physikalische Eigenschaften |
|---|---|---|---|---|---|---|
| I-56 | H | CN | $CH_3$<br>\|<br>$-CH-CO-NH-(CH_2)_3-CH_3$ | O | 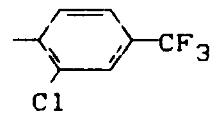 | Fp. 87° -94° C |
| I-57 | H | CN | $CH_3$<br>\|<br>$-CH-CO-NH-CH_3$ | O | 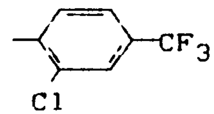 | Fp. 122° -129° C |
| I-58 | H | CN | $CH_3$<br>\|<br>$-CH-CO-NH-C_2H_5$ | O | 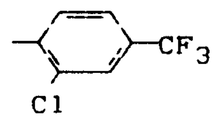 | Fp. 112-117° C |
| I-59 | H | CN | $CH_3$<br>\|<br>$-CH-COO-(CH_2)_2-CH_3$ | O | 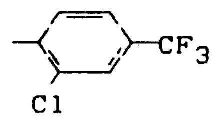 | $^1$H-NMR*): 7.9; 5.4 |
| I-60 | H | CN | $CH_3$<br>\|<br>$-CH-CO-NH-CH_2-CH=CH_2$ | O | 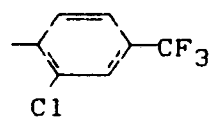 | Fp. 88° -91° C |

Tabelle 2 (Fortsetzung)

| Bsp. Nr. | $R^1$ | $R^2$ | $R^3$ | Y | Ar | physikalische Eigenschaften |
|---|---|---|---|---|---|---|
| I-61 | $(CH_3)_3C-$ | $NO_2$ | $CH_3$ | S | 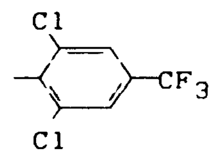 | $^1H$-NMR*): 7.78; |
| I-62 | $(CH_3)_3C-$ | $NO_2$ | $C_2H_5$ | O | 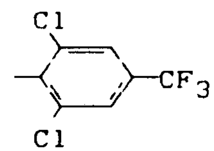 | $^1H$-NMR*): 7.76 |
| I-63 | $F_3C-$ | $NO_2$ | $C_2H_5$ | O | 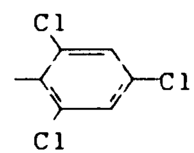 | Fp. 112° -115° C |

*) Die $^1H$-NMR-Spektren werden in $CDCl_3$ mit Tetramethylsilan als innerem Standard aufgenommen. Angegeben ist die chemische Verschiebung als δ-Wert in ppm.

Entsprechend dem Herstellungsbeispiel 2 und gemäß den allgemeinen Angaben zur Herstellung erhält man die folgenden Vorprodukte der allgemeinen Formel (IV)

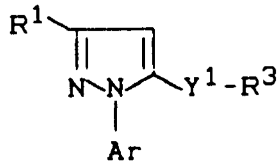

(IV)

Tabelle 3

| Bsp. Nr. | $R^1$ | $R^3$ | $Y^1$ | Ar | Fp (°C) bzw. $^1$HNMR in $CDCl_3$ in ppm |
|---|---|---|---|---|---|
| IV-2 | H | $CH_3$ | O | 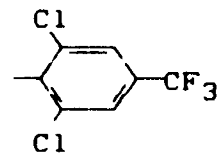 | 84-87 |
| IV-3 | H | $i-C_3H_7$ | O | 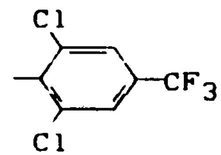 | 30-35 |
| IV-4 | H | $CH_3$<br>$-CH-COOCH_3$ | O | 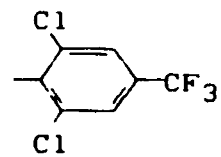 | 5,6(d,1H) 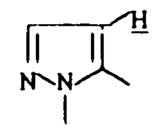<br>4,7(q,1H) $-O-C\underline{H}-$<br>$CH_3$ |
| IV-5 | H | $CH_3$<br>$-CH-COOC_2H_5$ | O | 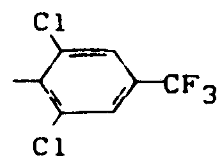 | 5,6(d,1H) 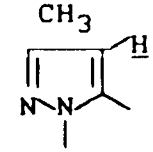<br>4,7(q,1H) $-O-C\underline{H}-$<br>$CH_3$ |

Tabelle 3 (Fortsetzung)

| Bsp. Nr. | $R^1$ | $R^3$ | $Y^1$ | Ar | Fp ($^oC$) bzw. $^1HNMR$ in $CDCl_3$ in ppm |
|---|---|---|---|---|---|
| IV-6 | H | $CH_3$<br>\|<br>$-CH-COOC_3H_7$ | O | 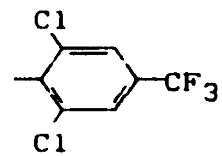 | 5,6(d,1H) 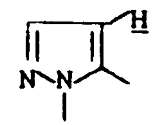<br>4,7(q,1H) -O-C<u>H</u>-<br>\|<br>$CH_3$ |
| IV-7 | H | $CH_3$<br>\|<br>$-CH-COOC_4H_9$ | O | 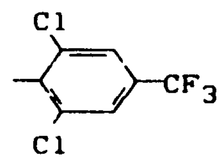 | 5,6(d,1H) 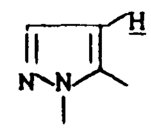<br>4,7(q,1H) -O-C<u>H</u>-<br>\|<br>$CH_3$ |
| IV-8 | H | $-CH_2-COOC_2H_5$ | O | 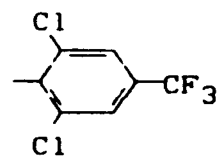 | 5,6(d,1H) 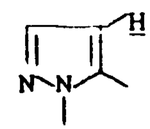<br>4,6(s,2H) $-O-C\underline{H}_2-$ |
| IV-9 | H | $CH_3$<br>\|<br>$-CH-COOCH_3$ | O | 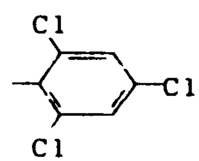 | 98 |

Tabelle 3 (Fortsetzung)

| Bsp. Nr. | $R^1$ | $R^3$ | $Y^1$ | Ar | Fp (°C) bzw. $^1HNMR$ in $CDCl_3$ in ppm |
|---|---|---|---|---|---|
| IV-10 | H | $CH_3$<br>\|<br>$-CH-COOC_2H_5$ | O | 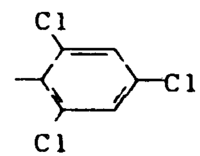 | 54 |
| IV-11 | H | $CH_3$<br>\|<br>$-CH-COOC_3H_7$ | O | 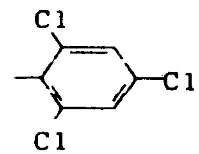 | 5,6(d,1H) 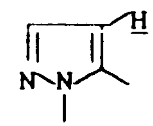<br>4,7(q,1H) $-O-C\underline{H}-$<br>\|<br>$CH_3$ |
| IV-12 | H | $CH_3$<br>\|<br>$-CH-COOC_4H_9$ | O | 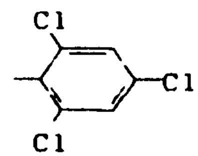 | 5.6(d,1H) 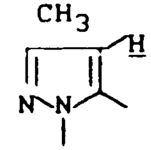<br>4,7(q,1H) $-O-C\underline{H}-$<br>\|<br>$CH_3$ |
| IV-13 | H | $-CH_2-COOC_2H_5$ | O | 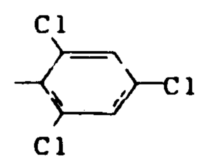 | 5,6(d,1H) 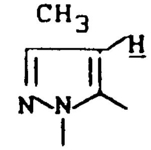<br>4,6(s,2H) $-O-C\underline{H}_2-$ |

Tabelle 3 (Fortsetzung)

| Bsp. Nr. | $R^1$ | $R^3$ | $Y^1$ | Ar | Fp (°C) bzw. $^1$HNMR in $CDCl_3$ in ppm |
|---|---|---|---|---|---|
| IV-14 | H | $CH_3$<br>\|<br>$-CH-COOCH_3$ | O | 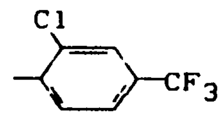 | 5,6(d,1H) 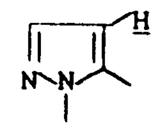<br>4,7(q,1H) -O-C<u>H</u>-<br>\|<br>$CH_3$ |
| IV-15 | H | $CH_3$<br>\|<br>$-CH-COOC_2H_5$ | O | 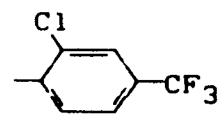 | 5,6(d,1H) 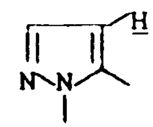<br>4,7(q,1H) -O-C<u>H</u>-<br>\|<br>$CH_3$ |
| IV-16 | H | $CH_3$<br>\|<br>$-CH-COOC_3H_7$ | O | 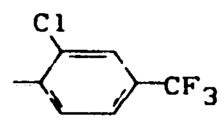 | 5,6(d,1H) 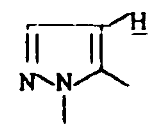<br>4,7(q,1H) -O-C<u>H</u>-<br>\|<br>$CH_3$ |

Tabelle 3 (Fortsetzung)

| Bsp. Nr. | $R^1$ | $R^3$ | $Y^1$ | Ar | Fp ($^0C$) bzw. $^1HNMR$ in $CDCl_3$ in ppm |
|---|---|---|---|---|---|
| IV-17 | H | $CH_3$<br>\|<br>$-CH-COOC_4H_9$ | O | 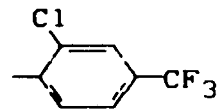 | 5,6(d,1H) 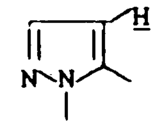<br>4,7(q,1H) -O-C$\underline{H}$-<br>\|<br>$CH_3$ |
| IV-18 | H | $-CH_2-COOC_2H_5$ | O | 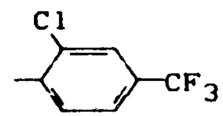 | 5,6(d,1H) 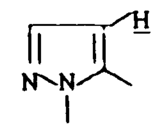<br>4,6(s,2H) -O-C$\underline{H}_2$- |
| IV-19 | H | $CH_3$<br>\|<br>$-CH-COOCH_3$ | O | 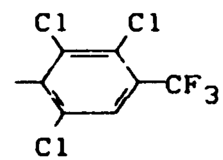 | 5,6(d,1H) 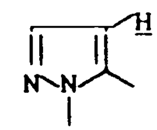<br>1,55(d,3H) -O-CH-<br>\|<br>C$\underline{H}_3$ |

Tabelle 3 (Fortsetzung)

| Bsp. Nr. | $R^1$ | $R^3$ | $Y^1$ | Ar | Fp (°C) bzw. $^1$HNMR in $CDCl_3$ in ppm |
|---|---|---|---|---|---|
| IV-20 | $CH_3$ | $CH_3$ | O | 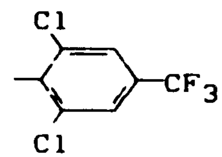 | 2,28(s,3H) 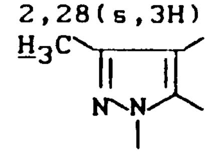 5,48(s,1H) 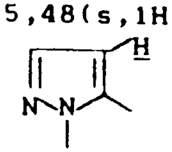 3,83(s,3H) 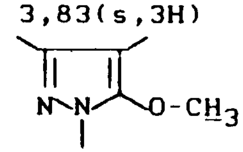 |
| IV-21 | $CH_3$ | $C_2H_5$ | O | 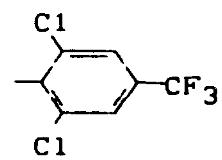 | $n_D^{23}$ = 1,5166 |
| IV-22 | $CH_3$ | i-$C_3H_7$ | O | 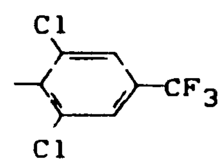 | $n_D^{23}$ =1,5093 |

Tabelle 3 (Fortsetzung)

| Bsp. Nr. | $R^1$ | $R^3$ | $Y^1$ | Ar | Fp (°C) bzw. $^1HNMR$ in $CDCl_3$ in ppm |
|---|---|---|---|---|---|
| IV-23 | H | 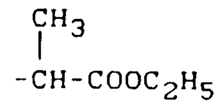 | O | 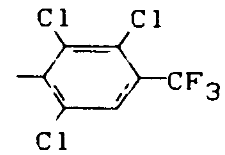 | 8,25(s,1H) 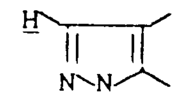<br>5,45(m,1H) 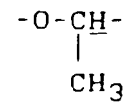 |
| IV-24 | H | 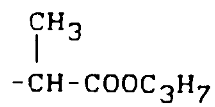 | O | 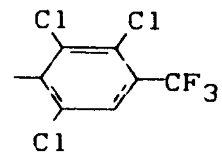 | 8,25(s,1H) 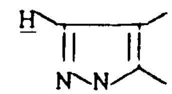<br>5,5 (m,1H) 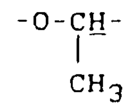 |
| IV-25 | H | $CH_3$ | S | 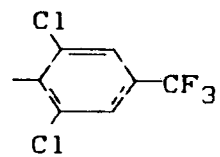 | 69 |

Herstellung der Vorprodukte der Formel (V)

Beispiel (V-1)

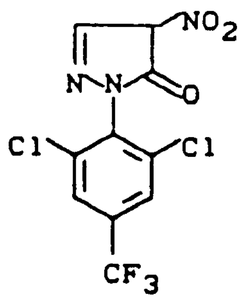

(V-1)

9 g (0,03 Mol) 1-(2,6-Dichlor-4-trifluormethyl-phenyl)-pyrazol-5-on werden fein gepulvert und portionsweise in eine Mischung aus 40 ml Wasser und 20 ml Salpetersäure (d = 1,5) eingetragen. Es wird noch über Nacht gerührt und das Festprodukt sodann abgesaugt, mit Wasser gewaschen und getrocknet. Zur Reinigung wird mit Ligroin verrieben, abgesaugt und getrocknet.
Man erhält 2,2 g (21 % der Theorie) 1-(2,6-Dichlor-4-trifluormethyl-phenyl)-4-nitro-pyrazol-5-on vom Schmelzpunkt 100-108 °C.

Beispiel (V-2)

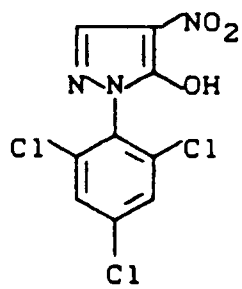

(V-2)

3,3 g 1-(2,4,6-Trichlorphenyl)-4-nitro-5-chlor-pyrazol werden in 10 ml Methanol suspendiert und 20 ml 1N, methanolische Kalilauge zugesetzt. Es wird 12 Stunden unter Rückfluß erhitzt. Zur vollständigen Umsetzung werden dem Reaktionsgemisch nochmals 10 ml 1N, methanolische Kalilauge zugesetzt und weitere 12 Stunden unter Rückfluß erhitzt. Zur Aufarbeitung wird filtriert, das Lösungsmittel im Vakuum abgezogen, der Rückstand mit heißem Wasser aufgenommen und das Produkt durch Ansäuern mit 2N Salzsäure ausgefällt. Nach Absaugen und Trocknen erhält man 1,5 g (49 % der Theorie) 1-(2,4,6-Trichlorphenyl)-4-nitro-5-hydroxy-pyrazol vom Schmelzpunkt 106-115 °C.

In entsprechender Weise und gemäß den allgemeinen Angaben zur Herstellung erhält man die folgenden Vorprodukte der allgemeinen Formel (V)

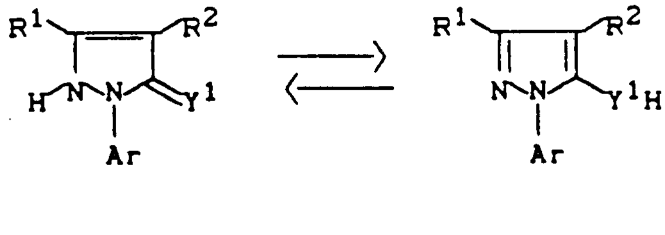

(V)

Tabelle 4

| Bsp.-Nr. | $R^1$ | $R^2$ | $Y^1$ | Ar | Fp(°C) |
|---|---|---|---|---|---|
| V-3 | H | CN | O | 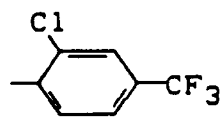 | 210-13 |

Anwendungsbeispiele

In den folgenden Anwendungsbeispielen wurde die nachstehend aufgeführte Verbindung als Vergleichssubstanz eingesetzt:

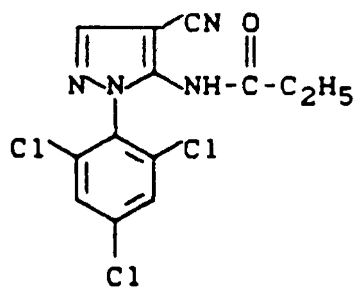

(A)

(bekannt aus DE-OS 32 26 513)

Beispiel A

Pre-emergence-Test

Lösungsmittel : 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Nutzpflanzenselektivität, insbesondere in Weizen, Baumwolle und Mais, gegenüber der Vergleichssubstanz (A) zeigt in diesem Test z.B. die Verbindung gemäß dem Herstellungsbeispiel: I-6.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
O % = keine Wirkung (wie unbehandelte Kontrolle)
100 % = totale Vernichtung

Eine deutliche Überlegenheit in der Nutzpflanzenselektivität, insbesondere in Weizen und Gerste, gegenüber der Vergleichssubstanz (A) zeigt in diesem Test z.B. die Verbindung gemäß dem Herstellungsbeispiel: I-6

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monalaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5, Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
\+ + starkes Austrocknen der Blätter, starker Blattfall
\+ + + sehr starkes Austrocknen der Blätter, sehr starker Blattfall

Eine deutliche Wirksamkeit im Vergleich zur unbehandelten Kontrolle zeigen in diesem Test beispielsweise die Verbindungen gemäß den Herstellungsbeispielen I-11, I-16, I-22, I-23, I-25, I-26 , I-31, I-33, I-34, I-38 und I-39.

## Patentansprüche

**1.** 4-Cyano(nitro)-5-oxy(thio)-pyrazol-Derivate der Formel

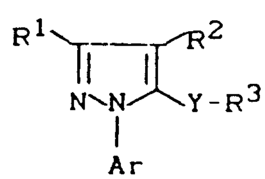

(I)

in welcher

$R^1$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^2$ für Nitro oder Cyano steht,

R³ für jeweils gegebenenfalls substituiertes, geradkettiges oder verzweigtes Alkyl, Alkenyl und Alkinyl mit jeweils bis zu 6 Kohlenstoffatomen steht, wobei als Substituenten jeweils genannt seien:
Halogen; Alkoxy, Alkylthio, Alkylsulfinyl, Alkylsulfonyl und Dialkoxy mit jeweils 1 bis 4 Kohlenstoffatomen in den Alkylteilen; Cyano; Hydroxycarbonyl; Alkoxycarbonyl, Alkylthiocarbonyl und Alkylcarbonyl mit jeweils 1 bis 4 Kohlenstoffatomen im Alkylteil; Aminocarbonyl; Alkylaminocarbonyl und Dialkylaminocarbonyl mit jeweils 1 bis 4 Kohlenstoffatomen in den Alkylteilen; Hydroximino und Alkoximino mit 1 bis 4 Kohlenstoffatomen;
sowie die Gruppierung

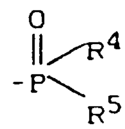

wobei

$R^4$ und $R^5$ gleich oder verschieden sind und für Hydroxy, Alkyl und Alkoxy mit jeweils 1 bis 4 Kohlenstoffatomen, sowie für jeweils gegebenenfalls einfach oder mehrfach, gleich oder verschieden substituiertes Phenyl und Phenoxy stehen, wobei als Substituenten vorzugsweise die für Ar als Phenylsubstituenten infrage kommenden Reste genannt seien;
ferner für jeweils gegebenenfalls einfach oder mehrfach, gleich oder verschieden substituiertes Cycloalkyl und Cycloalkylalkyl mit 3 bis 7 Kohlenstoff-atomen im Cycloalkylteil und 1 bis 2 Kohlenstoffatomen im Alkylteil steht, wobei als Substituenten im Cycloalkylteil jeweils genannt seien: Halogen, Alkyl, Alkoxy und Alkylthio mit jeweils 1 bis 4 Kohlenstoffatomen;
oder für jeweils gegebenenfalls einfach oder mehrfach, gleich oder verschieden substituiertes Phenyl und Phenylalkyl mit 1 bis 4 Kohlenstoffatomen im geradkettigen oder verzweigten Alkylteil steht, wobei als Phenylsubstituenten jeweils infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen;

Y für O, S, SO oder $SO_2$ steht und

Ar für zweifach oder höher substituiertes Phenyl sowie für jeweils gegebenenfalls einfach oder mehrfach, gleich oder verschieden substituiertes 2-Pyridyl, 3-Pyridyl oder 4-Pyridyl steht, wobei als Phenyl- bzw. Pyridyl-Substituenten in Frage kommen:
Cyano, Nitro, Halogen, jeweils geradkettiges oder verzweigtes Alkyl, Alkoxy oder Alkoxycarbonyl mit jeweils bis zu 4 Kohlenstoffatomen außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_mR^6$
wobei

$R^6$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für einen Zahl 0, 1 oder 2 steht.

**2.** 4-Cyano(nitro)-5-oxy(thio)-pyrazol-Derivate der Formel (I), gemäß Anspruch 1, in welcher

$R^1$ für Wasserstoff, Methyl, Ethyl, n- oder i-Propyl und Trifluormethyl steht;

$R^2$ für Nitro oder Cyano steht;

$R^3$ für Methyl, Ethyl; n- oder i-Propyl; n-, i-, s- oder t-Butyl; n- oder i-Pentyl; n- oder i-Hexyl, Allyl, Propenyl, Butenyl, Propargyl oder Butinyl steht, die jeweils einfach oder mehrfach, gleich oder verschieden substituiert sein können durch Fluor, Chlor, Brom, Methoxy, Ethoxy, Methylthio, Ethylthio, Methylsulfinyl, Methylsulfonyl, Dimethoxy, Diethoxy, Cyano, Hydroxycarbonyl, Methoxycarbonyl, Ethoxycarbonyl, Propoxycarbonyl, Butoxycarbonyl, Aminocarbonyl, Methylaminocarbonyl, Dimethylaminocarbonyl, Methylthiocarbonyl, Methylcarbonyl, Hydroximino, Methoximino, Ethoximino, Phosphonoyl, Methyl-phosphinoyl, Dimethylphosphinoyl, Methyl-ethylphosphinoyl, Dimethylphosphonoyl sowie Diethylphosphonoyl;

ferner für Cyclopropyl, Cyclopentyl, Cyclohexyl, Cyclopropylmethyl, Cyclopentylmethyl oder Cyclohexylmethyl steht, die jeweils einfach oder zweifach, gleich oder verschieden durch Fluor, Chlor oder Methyl substituiert sein können;

oder für jeweils gegebenenfalls ein- bis dreifach, gleich oder verschieden substituiertes Phenyl, Benzyl oder Phenylethyl steht, wobei als Phenylsubstituenten in Frage kommen: Fluor, Chlor, Brom, Iod, Cyano, Nitro, Methyl, Ethyl, Methoxy, Methylthio, Trifluormethyl, Methylsulfinyl, Methylsulfonyl, Trifluormethoxy, Trifluormethylthio, Trifluormethylsulfinyl und Trifluormethylsulfonyl;

Y für O, S, SO oder $SO_2$ steht und

Ar für zweifach oder höher substituiertes Phenyl sowie für jeweils gegebenenfalls ein- bis vierfach, gleich oder verschieden substituiertes 3-Pyridyl, 3-Pyridyl oder 4-Pyridyl steht, wobei als Phenyl- bzw. Pyridyl-Substituenten in Frage kommen:

Cyano, Nitro, Fluor, Chlor, Brom, Iod, Methyl, Ethyl, n- und i-Propyl, n-, i-, s- und t-Butyl, Methoxy, Ethoxy, Methoxycarbonyl, Ethoxycarbonyl, Trifluormethyl, Trichlormethyl, Difluorchlormethyl, Dichlorfluormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_mR^6$,

wobei

$R^6$ für Amino, Methylamino, Ethylamino, Dimethylamino, Diethylamino, Fluordichlormethyl, Difluormethyl, Tetrafluorethyl, Trifluorchlorethyl, Trifluormethyl, Methyl oder Ethyl steht und

m für eine Zahl 0, 1 oder 2 steht.

**3.** Verfahren zur Herstellung von 4-Cyano(nitro)-5-oxy(thio)-pyrazol-Derivate der Formel (I)

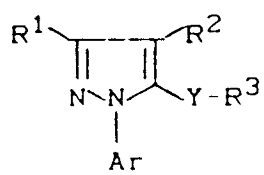

(I)

in welcher

$R^1$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^2$ für Nitro oder Cyano steht,

$R^3$ für jeweils gegebenenfalls substituiertes, geradkettiges oder verzweigtes Alkyl, Alkenyl und Alkinyl mit jeweils bis zu 6 Kohlenstoffatomen steht, wobei als Substituenten jeweils genannt seien:

Halogen; Alkoxy, Alkylthio, Alkylsulfinyl, Alkylsulfonyl und Dialkoxy mit jeweils 1 bis 4 Kohlenstoffatomen in den Alkylteilen; Cyano; Hydroxycarbonyl; Alkoxycarbonyl, Alkylthiocarbonyl und Alkylcarbonyl mit jeweils 1 bis 4 Kohlenstoffatomen im Alkylteil; Aminocarbonyl; Alkylaminocarbonyl und Dialkylaminocarbonyl mit jeweils 1 bis 4 Kohlenstoffatomen in den Alkylteilen; Hydroximino und Alkoximino mit 1 bis 4 Kohlenstoffatomen;

sowie die Gruppierung

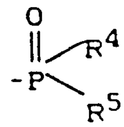

wobei

$R^4$ und $R^5$ gleich oder verschieden sind und für Hydroxy, Alkyl und Alkoxy mit jeweils 1 bis 4 Kohlenstoffatomen, sowie für jeweils gegebenenfalls einfach oder mehrfach, gleich oder verschieden substituiertes Phenyl und Phenoxy stehen, wobei als Substituenten vorzugsweise die für Ar als Phenylsubstituenten infrage kommenden Reste genannt seien;
ferner für jeweils gegebenenfalls einfach oder mehrfach, gleich oder verschieden substituiertes Cycloalkyl und Cycloalkylalkyl mit 3 bis 7 Kohlenstoff-atomen im Cycloalkylteil und 1 bis 2 Kohlenstoffatomen im Alkylteil steht, wobei als Substituenten im Cycloalkylteil jeweils genannt seien: Halogen, Alkyl, Alkoxy und Alkylthio mit jeweils 1 bis 4 Kohlenstoffatomen;
oder für jeweils gegebenenfalls einfach oder mehrfach, gleich oder verschieden substituiertes Phenyl und Phenylalkyl mit 1 bis 4 Kohlenstoffatomen im geradkettigen oder verzweigten Alkylteil steht, wobei als Phenylsubstituenten jeweils infrage kommen: Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen;

Y für O, S, SO oder $SO_2$ steht und

Ar für zweifach oder höher substituiertes Phenyl sowie für jeweils gegebenenfalls einfach oder mehrfach, gleich oder verschieden substituiertes 2-Pyridyl, 3-Pyridyl oder 4-Pyridyl steht, wobei als Phenyl- bzw. Pyridyl-Substituenten in Frage kommen: Cyano, Nitro, Halogen, jeweils geradkettiges oder verzweigtes Alkyl, Alkoxy oder Alkoxycarbonyl mit jeweils bis zu 4 Kohlenstoffatomen außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_mR^6$

wobei

$R^6$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für einen Zahl 0, 1 oder 2 steht,

dadurch gekennzeichnet, daß man

a) 4-Cyano(nitro)-5-oxy(thio)-pyrazol-Derivate der Formel (I) erhält,

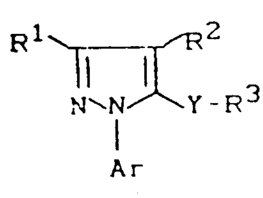

(I)

in welcher

$R^1$, $R^2$, $R^3$, Y und Ar die oben angegebene Bedeutung haben

wenn man 5-Halogen-pyrazol-Derivate der Formel (II)

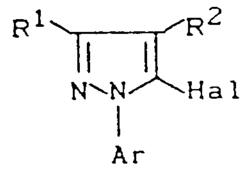

(II)

in welcher

$R^1$, $R^2$ und Ar die oben angegebene Bedeutung haben und

Hal für Fluor, Chlor oder Brom steht,

mit nucleophilen Verbindungen der Formel (III)

H – Y – $R^3$ (III)

in welcher

$R^3$ und Y die oben angegebene Bedeutung haben,

in Gegenwart eines Verdünnungsmittels und gegebenenfalls in Gegenwart eines Säurebindemittels umsetzt, oder daß man

b) 4 – Nitro – 5 – oxy(thio) – pyrazol – Derivate der Formel (Ia) erhält

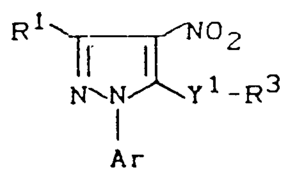

(Ia)

in welcher

$R^1$, $R^3$ und Ar die oben angegebene Bedeutung haben und

$Y^1$ für Sauerstoff oder Schwefel steht,

wenn man 5 – Oxy(thio) – pyrazol – Derivate der Formel (IV)

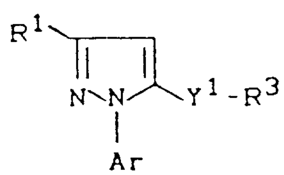

(IV)

in welcher

$R^1$, $R^3$, $Y^1$ und Ar die oben angegebene Bedeutung haben,

mit einem Nitrierungsmittel gegebenenfalls in Gegenwart eines Verdünnungsmittels und gegebenenfalls in Gegenwart eines Katalysators umsetzt,

oder, daß man

c) 4 – Cyano(nitro) – 5 – oxy(thio) – pyrazol – Derivate der Formel (Ib) erhält,

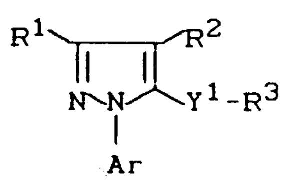

(Ib)

in welcher

$R^1$, $R^2$, $R^3$, $Y^1$ und Ar die oben angegebene Bedeutung haben,

wenn man Pyrazolin – one(thione) der Formel (V)

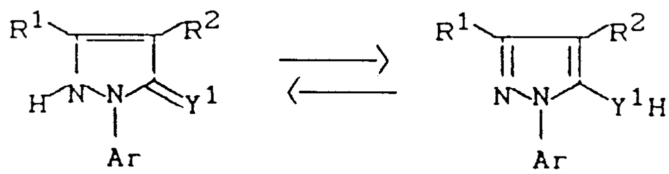

(V)

in welcher

$R^1$, $R^2$, Ar und $Y^1$ die oben angegebene Bedeutung haben,

mit Alkylierungsmitteln der Formel (VI)

A − $R^3$ (VI)

worin

$R^3$ die oben angegebene Bedeutung hat und

A für eine elektronenziehende Abgangsgruppe steht,

gegebenenfalls in Gegenwart eines Verdünnungsmittels und gegebenenfalls in Gegenwart eines Säurebindemittels umsetzt, oder daß man

d) 5 − Sulfinyl(sulfonyl) − pyrazol − Derivate der Formel (Ic) erhält

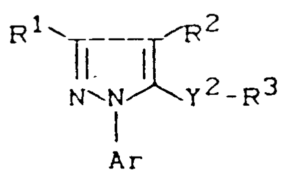

(Ic)

in welcher

$R^1$, $R^2$, $R^3$ und Ar die oben angegebene Bedeutung haben

und

$Y^2$ für SO oder $SO_2$ steht,

wenn man 5 − Thio − pyrazol − Derivate der Formel (Id)

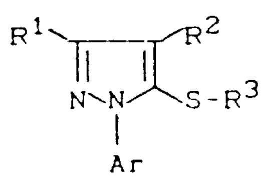

(Id)

in welcher

$R^1$, $R^2$, $R^3$ und Ar die oben angegebene Bedeutung haben,

oxidiert, oder daß man

e) 4 − Cyano(nitro) − 5 − oxy(thio) − pyrazol − Derivate der Formel (Ib) erhält,

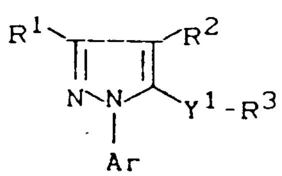

(Ib)

in welcher

$R^1$, $R^2$, $R^3$, $Y^1$ und Ar die oben angegebene Bedeutung haben,

wenn man 5 − Sulfonyl − pyrazol − Derivate der Formel (Ie)

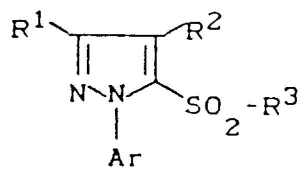

(Ie)

in welcher

$R^1$, $R^2$, $R^3$ und Ar die oben angegebene Bedeutung haben,

mit nucleophilen Verbindungen der Formel (IIIa)

$H - Y^1 - R^3$ (IIIa)

in welcher $R^3$ und $Y^1$ die oben angegebene Bedeutung haben, in Gegenwart eines Verdünnungsmittels und gegebenenfalls in Gegenwart eines Säurebindemittels umsetzt.

**4.** Herbizide und pflanzenwuchsregulierende Mittel, gekennzeichnet durch einen Gehalt an mindestens einem 4-Cyano(nitro)-5-oxy(thio)-pyrazol-Derivat der Formel (I) gemäß den Ansprüchen 1 bis 3.

**5.** Verfahren zur Bekämpfung von Unkräutern, dadurch gekennzeichnet, daß man 4-Cyano(nitro)-5-oxy(thio)-pyrazol-Derivate der Formel (I) gemäß den Ansprüchen 1 bis 3 auf die Unkräuter und/oder ihren Lebensraum einwirken läßt.

**6.** Verwendung von 4-Cyano(nitro)-5-oxy(thio)-pyrazol-Derivaten der Formel (I) gemäß den Ansprüchen 1 bis 3 zur Bekämpfung von Unkräutern und/oder als Pflanzenwachstumsregulatoren.

**7.** Verfahren zur Herstellung von herbiziden und/oder pflanzenwuchsregulierenden Mitteln, dadurch gekennzeichnet, daß man 4-Cyano(nitro)-5-oxy(thio)-pyrazol-Derivate der Formel (I) gemäß den Ansprüchen 1 bis 3 mit Streckmitteln und/oder oberflächenaktiven Substanzen vermischt.

**8.** 5-Oxy(thio)-pyrazol-Derivate der Formel (IV)

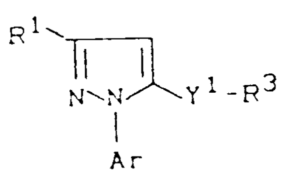

(IV)

in welcher

$R^1$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^3$ für jeweils gegebenenfalls substituiertes, geradkettiges oder verzweigtes Alkyl, Alkenyl und Alkinyl mit jeweils bis zu 6 Kohlenstoffatomen steht, wobei als Substituenten jeweils genannt seien:

Halogen; Alkoxy, Alkylthio, Alkylsulfinyl, Alkylsulfonyl und Dialkoxy mit jeweils 1 bis 4 Kohlenstoffatomen in den Alkylteilen; Cyano; Hydroxycarbonyl; Alkoxycarbonyl, Alkylthiocarbonyl und Alkylcarbonyl mit jeweils 1 bis 4 Kohlenstoffatomen im Alkylteil; Aminocarbonyl; Alkylaminocarbonyl und Dialkylaminocarbonyl mit jeweils 1 bis 4 Kohlenstoffatomen in den Alkylteilen; Hydroximino und Alkoximino mit 1 bis 4 Kohlenstoffatomen;

sowie die Gruppierung

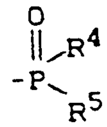,

wobei

$R^4$ und $R^5$ gleich oder verschieden sind und für Hydroxy, Alkyl und Alkoxy mit jeweils 1 bis 4 Kohlenstoffatomen, sowie für jeweils gegebenenfalls einfach oder mehrfach, gleich oder verschieden substituiertes Phenyl und Phenoxy stehen, wobei als Substituenten vorzugsweise die für Ar als Phenylsubstituenten infrage kommenden Reste genannt seien;

ferner für jeweils gegebenenfalls einfach oder mehrfach, gleich oder verschieden substituiertes Cycloalkyl und Cycloalkylalkyl mit 3 bis 7 Kohlenstoffatomen im Cycloalkylteil und 1 bis 2 Kohlenstoffatomen im Alkylteil steht, wobei als Substituenten im Cycloalkylteil jeweils genannt seien: Halogen, Alkyl, Alkoxy und Alkylthio mit jeweils 1 bis 4 Kohlenstoffatomen;

oder für jeweils gegebenenfalls einfach oder mehrfach, gleich oder verschieden substituiertes Phenyl und Phenylalkyl mit 1 bis 4 Kohlenstoffatomen im geradkettigen oder verzweigten Alkylteil steht, wobei als Phenylsubstituenten jeweils infrage kommen:

Halogen, Cyano, Nitro, jeweils geradkettiges oder verzweigtes Alkyl, Alkoxy, Alkylthio, Alkylsulfinyl, Alkylsulfonyl, Halogenalkyl, Halogenalkoxy, Halogenalkylthio, Halogenalkylsulfinyl oder Halogenalkylsulfonyl mit jeweils 1 bis 4 Kohlenstoffatomen in den einzelnen Alkylteilen und gegebenenfalls 1 bis 9 gleichen oder verschiedenen Halogenatomen;

$Y^1$ für Sauerstoff oder Schwefel steht und

Ar für zweifach oder höher substituiertes Phenyl sowie für jeweils gegebenenfalls einfach oder mehrfach, gleich oder verschieden substituiertes 2-Pyridyl, 3-Pyridyl oder 4-Pyridyl steht, wobei als Phenyl- bzw. Pyridyl-Substituenten in Frage kommen:

Cyano, Nitro, Halogen, jeweils geradkettiges oder verzweigtes Alkyl, Alkoxy oder Alkoxycarbonyl mit jeweils bis zu 4 Kohlenstoffatomen außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_mR^6$

wobei

$R^6$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für eine Zahl 0, 1 oder 2 steht.

**9.** Pyrazolin-one(thione) der Formel (V)

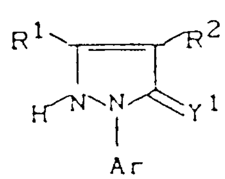

(V)

in welcher

$R^1$ für Wasserstoff, für geradkettiges oder verzweigtes Alkyl mit 1 bis 4 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^2$ für Nitro oder Cyano steht,

$Y^1$ für Sauerstoff oder Schwefel steht und

Ar für zweifach oder höher substituiertes Phenyl sowie für jeweils gegebenenfalls einfach oder

mehrfach, gleich oder verschieden substituiertes 2-Pyridyl, 3-Pyridyl oder 4-Pyridyl steht, wobei als Phenyl- bzw. Pyridyl-Substituenten in Frage kommen:
Cyano, Nitro, Halogen, jeweils geradkettiges oder verzweigtes Alkyl, Alkoxy oder Alkoxycarbonyl mit jeweils bis zu 4 Kohlenstoffatomen außerdem jeweils geradkettiges oder verzweigtes Halogenalkyl oder Halogenalkoxy mit jeweils bis zu 4 Kohlenstoffatomen und bis zu 9 gleichen oder verschiedenen Halogenatomen oder ein Rest $-S(O)_mR^6$
wobei

$R^6$ für Amino, sowie für jeweils geradkettiges oder verzweigtes Alkyl, Alkylamino, Dialkylamino oder Halogenalkyl mit jeweils bis zu 4 Kohlenstoffatomen in den einzelnen Alkylteilen und im Fall des Halogenalkyl mit bis zu 9 gleichen oder verschiedenen Halogenatomen steht und

m für eine Zahl 0, 1 oder 2 steht.

## Claims

**1.** 4-Cyano(nitro)-5-oxy(thio)-pyrazole derivatives of the formula

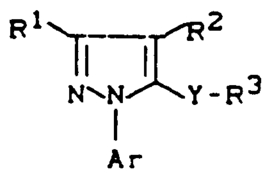

(I)

in which

$R^1$ represents hydrogen, straight-chain or branched alkyl having 1 to 4 carbon atoms, or straight-chain or branched halogenoalkyl having 1 to 4 carbon atoms and 1 to 9 identical or different halogen atoms,

$R^2$ represents nitro or cyano,

$R^3$ represents in each case optionally substituted, straight-chain or branched alkyl, alkenyl and alkinyl in each case having up to 6 carbon atoms, where the following may be mentioned in each case as substituents:
halogen; alkoxy, alkylthio, alkylsulphinyl, alkylsulphonyl and dialkoxy in each case having 1 to 4 carbon atoms in the alkyl parts; cyano; hydroxycarbonyl; alkoxycarbonyl, alkylthiocarbonyl and alkylcarbonyl in each case having 1 to 4 carbon atoms in the alkyl part; aminocarbonyl; alkylaminocarbonyl and dialkylaminocarbonyl in each case having 1 to 4 carbon atoms in the alkyl parts;
hydroximino and alkoximino having 1 to 4 carbon atoms; and the grouping

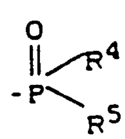

where

$R^4$ and $R^5$ are identical or different and represent hydroxyl, alkyl and alkoxy in each case having 1 to 4 carbon atoms, and also phenyl and phenoxy, which are in each case optionally mono- or polysubstituted, where the substituents are identical or different and where the radicals which are suitable as phenyl substituents for Ar are preferably mentioned as substituents;
furthermore represents cycloalkyl and cycloalkylalkyl, having 3 to 7 carbon atoms in the cycloalkyl part and 1 to 2 carbon atoms in the alkyl part, which are in each case optionally mono- or polysubstituted, where the substituents are identical or different and where the following may be mentioned in each case as substituents in the cycloalkyl part: halogen, alkyl, alkoxy and alkylthio in each case having 1 to 4 carbon atoms;
or represents phenyl and phenylalkyl, having 1 to 4 carbon atoms in the straight-

chain or branched alkyl part, which are in each case optionally mono- or polysubstituted, where the substituents are identical or different and suitable phenyl substituents in each case being: halogen, cyano, nitro, in each case straight-chain or branched alkyl, alkoxy, alkylthio, alkylsulphinyl, alkylsulphonyl, halogenoalkyl, halogenoalkoxy, halogenoalkylthio, halogenoalkylsulphinyl or halogenoalkylsulphonyl in each case having 1 to 4 carbon atoms in the individual alkyl parts and, if appropriate, 1 to 9 identical or different halogen atoms;

Y represents O, S, SO or $SO_2$ and

Ar represents phenyl which is substituted twice or more, and 2-pyridyl, 3-pyridyl or 4-pyridyl which are in each case optionally mono- or polysubstituted, where the substituents are identical or different and where the following are suitable as phenyl or pyridyl substituents:

cyano, nitro, halogen, in each case straight-chain or branched alkyl, alkoxy or alkoxycarbonyl in each case having up to 4 carbon atoms, in addition in each case straight-chain or branched halogenoalkyl or halogenoalkoxy in each case having up to 4 carbon atoms and up to 9 identical or different halogen atoms, or a $-S(O)_mR^6$ radical

where

$R^6$ represents amino, and also in each case straight-chain or branched alkyl, alkylamino, dialkylamino or halogenoalkyl in each case having up to 4 carbon atoms in the individual alkyl parts and, in the case of halogenoalkyl, having up to 9 identical or different halogen atoms, and

m represents a number 0, 1 or 2.

**2.** 4-Cyano(nitro)-5-oxy(thio)-pyrazole derivatives of the formula (I), according to Claim 1, in which

$R^1$ represents hydrogen, methyl, ethyl, n- or i-propyl and trifluoromethyl;

$R^2$ represents nitro or cyano;

$R^3$ represents methyl, ethyl; n- or i-propyl; n-, i-, s- or t-butyl; n- or i-pentyl; n- or i-hexyl, allyl, propenyl, butenyl, propargyl or butinyl, which may in each case be mono- or polysubstituted, where the substituents are identical or different, by fluorine, chlorine, bromine, methoxy, ethoxy, methylthio, ethylthio, methylsulphinyl, methylsulphonyl, dimethoxy, diethoxy, cyano, hydroxycarbonyl, methoxycarbonyl, ethoxycarbonyl, propoxycarbonyl, butoxycarbonyl, aminocarbonyl, methylaminocarbonyl, dimethylaminocarbonyl, methylthiocarbonyl, methylcarbonyl, hydroximino, methoximino, ethoximino, phosphonoyl, methylphosphinoyl, dimethylphosphinoyl, methyl-ethylphosphinoyl, dimethylphosphonoyl and diethylphosphonoyl;

furthermore represents cyclopropyl, cyclopentyl, cyclohexyl, cyclopropylmethyl, cyclopentylmethyl or cyclohexylmethyl, which may in each case be mono- or disubstituted, where the substituents are identical or different, by fluorine, chlorine or methyl;

or represents phenyl, benzyl or phenylethyl, which are in each case optionally mono- to trisubstituted, where the substituents are identical or different, suitable phenyl substituents being: fluorine, chlorine, bromine, iodine, cyano, nitro, methyl, ethyl, methoxy, methylthio, trifluoromethyl, methylsulphinyl, methylsulphonyl, trifluoromethoxy, trifluoromethylthio, trifluoromethylsulphinyl and trifluoromethylsulphonyl;

Y represents O, S, SO or $SO_2$ and

Ar represents phenyl which is substituted twice or more, and also 2-pyridyl, 3-pyridyl or 4-pyridyl, which are in each case optionally mono- to tetra-substituted, where the substituents are identical or different, suitable phenyl or pyridyl substituents being: cyano, nitro, fluorine, chlorine, bromine, iodine, methyl, ethyl, n- and i-propyl, n-, i-, s- and t-butyl, methoxy, ethoxy, methoxycarbonyl, ethoxycarbonyl, trifluoromethyl, trichloromethyl, difluorochloromethyl, dichlorofluoromethyl, chloromethyl, dichloromethyl, difluoromethyl, pentafluoroethyl, tetrafluoroethyl, trifluorochloroethyl, trifluoroethyl, difluorodichloroethyl, trifluorodichloroethyl, pentachloroethyl, trifluoromethoxy, trichloromethoxy, dichlorofluoromethoxy, difluorochloromethoxy, chloromethoxy, dichloromethoxy, difluoromethoxy, pentafluoroethoxy, tetrafluoroethoxy, trifluorochloroethoxy, trifluoroethoxy, difluorodichloroethoxy, trifluorodichloroethoxy, pentachloroethoxy or an $-S(O)_mR^6$ radical,

where

$R^6$ represents amino, methylamino, ethylamino, dimethylamino, diethylamino, fluorodich-

loromethyl, difluoromethyl, tetrafluoroethyl, trifluorochloroethyl, trifluoromethyl, methyl or ethyl and

m represents a number 0, 1 or 2.

**3.** Process for the preparation of 4-cyano(nitro)-5-oxy(thio)-pyrazole derivatives of the formula (I)

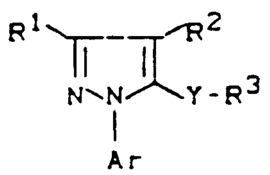

(I)

in which

$R^1$ represents hydrogen, straight-chain or branched alkyl having 1 to 4 carbon atoms, or straight-chain or branched halogenoalkyl having 1 to 4 carbon atoms and 1 to 9 identical or different halogen atoms,

$R^2$ represents nitro or cyano,

$R^3$ represents in each case optionally substituted, straight-chain or branched alkyl, alkenyl and alkinyl in each case having up to 6 carbon atoms, where the following may be mentioned in each case as substituents:

halogen; alkoxy, alkylthio, alkylsulphinyl, alkylsulphonyl and dialkoxy in each case having 1 to 4 carbon atoms in the alkyl parts; cyano; hydroxycarbonyl; alkoxycarbonyl, alkylthiocarbonyl and alkylcarbonyl in each case having 1 to 4 carbon atoms in the alkyl part; aminocarbonyl; alkylaminocarbonyl and dialkylaminocarbonyl in each case having 1 to 4 carbon atoms in the alkyl parts; hydroximino and alkoximino having 1 to 4 carbon atoms; and the grouping

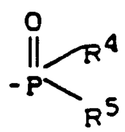

where

$R^4$ and $R^5$ are identical or different and represent hydroxyl, alkyl and alkoxy in each case having 1 to 4 carbon atoms, and also phenyl and phenoxy, which are in each case optionally mono- or polysubstituted, where the substituents are identical or different and where the radicals which are suitable as phenyl substituents for Ar are preferably mentioned as substituents;

furthermore represents cycloalkyl and cycloalkylalkyl, having 3 to 7 carbon atoms in the cycloalkyl part and 1 to 2 carbon atoms in the alkyl part, which are in each case optionally mono- or polysubstituted, where the substituents are identical or different and where the following may be mentioned in each case as substituents in the cycloalkyl part: halogen, alkyl, alkoxy and alkylthio in each case having 1 to 4 carbon atoms;

or represents phenyl and phenylalkyl, having 1 to 4 carbon atoms in the straight-chain or branched alkyl part, which are in each case optionally mono- or polysubstituted, where the substituents are identical or different and suitable phenyl substituents in each case being: halogen, cyano, nitro, in each case straight-chain or branched alkyl, alkoxy, alkylthio, alkylsulphinyl, alkylsulphonyl, halogenoalkyl, halogenoalkoxy, halogenoalkylthio, halogenoalkylsulphinyl or halogenoalkylsulphonyl in each case having 1 to 4 carbon atoms in the individual alkyl parts and, if appropriate, 1 to 9 identical or different halogen atoms;

Y represents O, S, SO or $SO_2$ and

Ar represents phenyl which is substituted twice or more, and 2-pyridyl, 3-pyridyl or 4-pyridyl which are in each case optionally mono- or polysubstituted, where the substituents are identical or different and where the following are suitable as phenyl or pyridyl substituents:

cyano, nitro, halogen, in each case straight-chain or branched alkyl, alkoxy or alkoxycarbonyl in each case having up to 4 carbon atoms, in addition in each case straight-chain or branched halogenoalkyl or halogenoalkoxy in each case having up to 4 carbon atoms and up to 9 identical or different halogen atoms, or a $-S(O)_mR^6$ radical
where

$R^6$ represents amino, and also in each case straight-chain or branched alkyl, alkylamino, dialkylamino or halogenoalkyl in each case having up to 4 carbon atoms in the individual alkyl parts and, in the case of halogenoalkyl, having up to 9 identical or different halogen atoms, and

m represents a number 0, 1 or 2.

characterised in that

a) 4-cyano(nitro)-5-oxy(thio)-pyrazole derivatives of the formula (I)

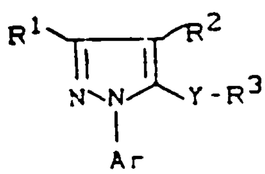

(I)

in which
$R^1$, $R^2$, $R^3$, Y and Ar have the abovementioned meaning, are obtained when 5-halogeno-pyrazole derivatives of the formula (II)

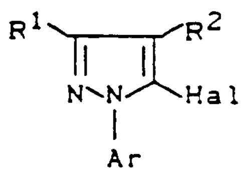

(II)

in which
$R^1$, $R^2$ and Ar have the abovementioned meaning and Hal represents fluorine, chlorine or bromine, are reacted with nucleophilic compounds of the formula (III)

$H - Y - R^3$ (III)

in which
$R^3$ and Y have the abovementioned meaning,
in the presence of a diluent and if appropriate in the presence of an acid-binding agent, or in that
b) 4-nitro-5-oxy(thio)-pyrazole derivatives of the formula (Ia)

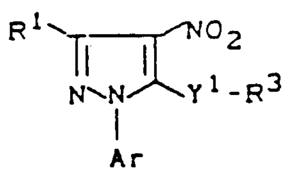

(Ia)

in which
$R^1$, $R^3$ and Ar have the abovementioned meaning and $Y^1$ represents oxygen or sulphur,
are obtained when 5-oxy(thio)-pyrazole derivatives of the formula (IV)

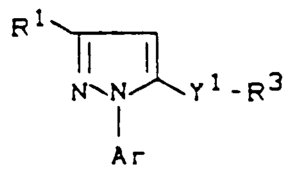

(IV)

in which

$R^1$, $R^3$, $Y^1$ and Ar have the abovementioned meaning, are reacted with a nitrating agent, if appropriate in the presence of a diluent and if appropriate in the presence of a catalyst,

or in that

c) 4 – cyano(nitro)5 – oxy(thio) – pyrazole derivatives of the formula (Ib)

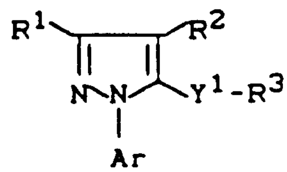

(Ib)

in which

$R^1$, $R^2$, $R^3$, $Y^1$ and Ar have the abovementioned meaning, are obtained when pyrazolinones or pyrazolinethiones of the formula (V)

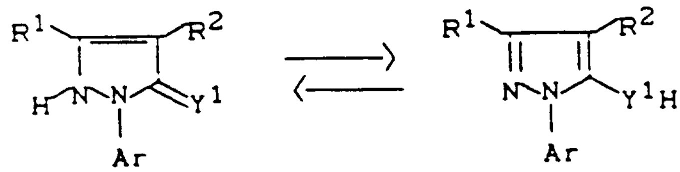

(V)

in which

$R^1$, $R^2$, Ar and $Y^1$ have the abovementioned meaning, are reacted with alkylating agents of the formula (VI)

$A - R^3$ (VI)

in which

$R^3$ has the abovementioned meaning and

A represents an electron – withdrawing leaving group,

if appropriate in the presence of a diluent and if appropriate in the presence of an acid – binding agent, or in that

d) 5 – sulphinyl(sulphonyl) – pyrazole derivatives of the formula (Ic)

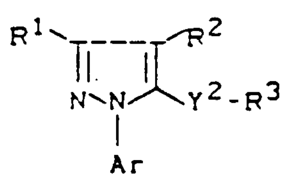

(Ic)

in which

$R^1$, $R^2$, $R^3$ and Ar have the abovementioned meaning and $Y^2$ represents SO or $SO_2$,

are obtained when 5 – thio – pyrazole derivatives of the formula (Id)

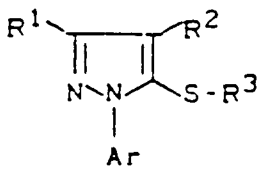

(Id)

in which

$R^1$, $R^2$, $R^3$ and Ar have the abovementioned meaning, are oxidised, or in that

e) 4-cyano(nitro)-5-oxy(thio)-pyrazole derivatives of the formula (Ib)

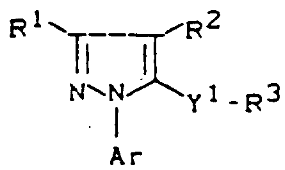

(Ib)

in which

$R^1$, $R^2$, $R^3$, $Y^1$ and Ar have the abovementioned meaning, are obtained when 5-sulphonyl-pyrazole derivatives of the formula (Ie)

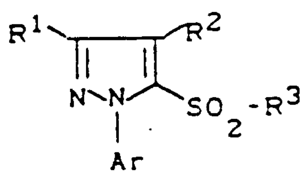

(Ie)

in which

$R^1$, $R^2$, $R^3$ and Ar have the abovementioned meaning, are reacted with nucleophilic compounds of the formula (IIIa)

$H - Y^1 - R^3$ (IIIa)

in which

$R^3$ and $Y^1$ have the abovementioned meaning,

in the presence of a diluent and if appropriate in the presence of an acid-binding agent.

**4.** Herbicidal and plant growth-regulating agents, characterised in that they contain at least one 4-cyano(nitro)-5-oxy(thio)-pyrazole derivative of the formula (I) according to Claims 1 to 3.

**5.** Process for combating weeds, characterised in that 4-cyano(nitro)-5-oxy(thio)-pyrazole derivatives of the formula (I) according to Claims 1 to 3 are allowed to act on the weeds and/or their habitat.

**6.** Use of 4-cyano(nitro)-5-oxy(thio)-pyrazole derivatives of the formula (I) according to Claims 1 to 3 for combating weeds and/or as plant growth regulators.

**7.** Process for the preparation of herbicidal and/or plant growth-regulating agents, characterised in that 4-cyano(nitro)-5-oxy(thio)-pyrazole derivatives of the formula (I) according to Claims 1 to 3 are mixed with extenders and/or surface-active substances.

**8.** 5 – Oxy(thio) – pyrazole derivatives of the formula (IV)

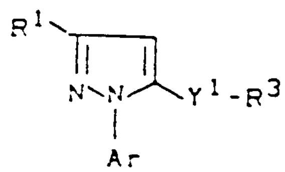

(IV)

in which

R¹ represents hydrogen, straight – chain or branched alkyl having 1 to 4 carbon atoms, or straight – chain or branched halogenoalkyl having 1 to 4 carbon atoms and 1 to 9 identical or different halogen atoms,

R³ represents in each case optionally substituted, straight – chain or branched alkyl, alkenyl and alkinyl in each case having up to 6 carbon atoms, where the following may be mentioned in each case as substituents:
halogen; alkoxy, alkylthio, alkylsulphinyl, alkylsulphonyl and dialkoxy in each case having 1 to 4 carbon atoms in the alkyl parts; cyano; hydroxycarbonyl; alkoxycarbonyl, alkylthiocarbonyl and alkylcarbonyl in each case having 1 to 4 carbon atoms in the alkyl part; aminocarbonyl; alkylaminocarbonyl and dialkylaminocarbonyl in each case having 1 to 4 carbon atoms in the alkyl parts; hydroximino and alkoximino having 1 to 4 carbon atoms; and the grouping

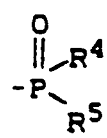

where

$R^4$ and $R^5$ are identical or different and represent hydroxyl, alkyl and alkoxy in each case having 1 to 4 carbon atoms, and also phenyl and phenoxy, which are in each case optionally mono – or polysubstituted, where the substituents are identical or different and where the radicals which are suitable as phenyl substituents for Ar are preferably mentioned as substituents;
furthermore represents cycloalkyl and cycloalkylalkyl, having 3 to 7 carbon atoms in the cycloalkyl part and 1 to 2 carbon atoms in the alkyl part, which are in each case optionally mono – or polysubstituted, where the substituents are identical or different and where the following may be mentioned in each case as substituents in the cycloalkyl part: halogen, alkyl, alkoxy and alkylthio in each case having 1 to 4 carbon atoms;
or represents phenyl and phenylalkyl, having 1 to 4 carbon atoms in the straight – chain or branched alkyl part, which are in each case optionally mono – or polysubstituted, where the substituents are identical or different and suitable phenyl substituents in each case being: halogen, cyano, nitro, in each case straight – chain or branched alkyl, alkoxy, alkylthio, alkylsulphinyl, alkylsulphonyl, halogenoalkyl, halogenoalkoxy, halogenoalkylthio, halogenoalkylsulphinyl or halogenoalkylsulphonyl in each case having 1 to 4 carbon atoms in the individual alkyl parts and, if appropriate, 1 to 9 identical or different halogen atoms;

$Y^1$ represents oxygen or sulphur and

Ar represents phenyl which is substituted twice or more, and 2 – pyridyl, 3 – pyridyl or 4 – pyridyl which are in each case optionally mono – or polysubstituted, where the substituents are identical or different and where the following are suitable as phenyl or pyridyl substituents:
cyano, nitro, halogen, in each case straight – chain or branched alkyl, alkoxy or alkoxycarbonyl in each case having up to 4 carbon atoms, in addition in each case straight – chain or branched halogenoalkyl or halogenoalkoxy in each case having up

to 4 carbon atoms and up to 9 identical or different halogen atoms, or a $-S(O)_mR^6$ radical
where

$R^6$ represents amino, and also in each case straight-chain or branched alkyl, alkylamino, dialkylamino or halogenoalkyl in each case having up to 4 carbon atoms in the individual alkyl parts and, in the case of halogenoalkyl, having up to 9 identical or different halogen atoms, and

m represents a number 0, 1 or 2.

**9.** Pyrazolinones and pyrazolinethiones of the formula (V)

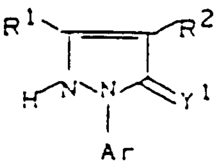

(V)

in which

$R^1$ represents hydrogen, straight-chain or branched alkyl having 1 to 4 carbon atoms, or straight-chain or branched halogenoalkyl having 1 to 4 carbon atoms and 1 to 9 identical or different halogen atoms,

$R^2$ represents nitro or cyano,

$Y^1$ represents oxygen or sulphur and

Ar represents phenyl which is substituted twice or more, and 2-pyridyl, 3-pyridyl or 4-pyridyl which are in each case optionally mono- or polysubstituted, where the substituents are identical or different and where the following are suitable as phenyl or pyridyl substituents:
cyano, nitro, halogen, in each case straight-chain or branched alkyl, alkoxy or alkoxycarbonyl in each case having up to 4 carbon atoms, in addition in each case straight-chain or branched halogenoalkyl or halogenoalkoxy in each case having up to 4 carbon atoms and up to 9 identical or different halogen atoms, or a $-S(O)_mR^6$ radical
where

$R^6$ represents amino, and also in each case straight-chain or branched alkyl, alkylamino, dialkylamino or halogenoalkyl in each case having up to 4 carbon atoms in the individual alkyl parts and, in the case of halogenoalkyl, having up to 9 identical or different halogen atoms, and

m represents a number 0, 1 or 2.

## Revendications

**1.** Dérivés de 4-cyano(nitro)-5-oxy(thio)-pyrazole, de formule :

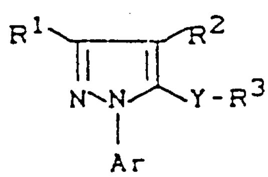

( I )

dans laquelle

$R^1$ représente un atome d'hydrogène, un coupe alkyle linéaire ou ramifié ayant 1 à 4 atomes de carbone ou un coupe halogénoalkyle, linéaire ou ramifié, ayant 1 à 4 atomes de carbone et 1 à 9 atomes d'halogène, identique-ou différents,

$R^2$ représente un coupe nitro ou cyano,

$R^3$ représente un coupe alkyle, alcényle ou alcynyle, linéaires ou ramifiés, chacun éventuellement substitué, et comportant chacun jusqu'à 4 atomes de carbone, et l'on peut alors citer comme substituants :
un atome d'halogène ; un reste alcoxy, alkythio, alkylsulfinyle, alkylsulfonyle et dialcoxy

ayant chacun 1 à 4 atomes d'halogène dans la partie alkyle ; un reste cyano ; hydroxycarbonyle ; alcoxycarbonyle, alkylthiocarbonyle et alkylcarbonyle ayant chacun 1 à 4 atomes de carbone dans la partie alkyle ; aminocarbonyle ; alkylaminocarbonyle et dialkylaminocarbonyle ayant chacun 1 à 4 atomes de carbone dans les parties alkyles ; hydroxyimino et alcoxyimino ayant 1 à 4 atomes de carbone,
ainsi que le groupe

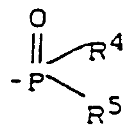

,

dans lequel

$R^4$ et $R^5$ sont identiques ou différents et représentent chacun un groupe hydroxy, alkyle et alcoxy ayant chacun 1 à 4 atomes de carbone, et un groupe phényle et phénoxy, éventuellement substitués une ou plusieurs fois, de façon identique ou différente, et l'on peut citer comme substituant, avantageusement les restes indiqués pour les substituants du groupe phényle ;

$R^3$ peut représenter en outre un groupe cycloalkyle et cycloalkylalkyle, identiques ou différents et chaque fois éventuellement substitués une ou plusieurs fois, le groupes ayant 3 à 7 atomes de carbone dans la partie cycloalkyle et 1 à 2 atomes de carbone dans la partie alkyle, et l'on peut citer comme substituant de la partie cycloalkyle : un atome d'halogène, un reste alkyle, alcoxy et alkylthio ayant chacun 1 à 4 atomes de carbone ;
ou un groupe phényle et phénylalkyle, éventuellement substitués une ou plusieurs de façon identique ou différente et comportant 1 à 4 atomes de carbone dans la partie alkyle linéaire ou ramifiée, et l'on peut citer comme substituant du noyau phényle, à chaque fois : un atome d'halogène, un reste cyano, nitro, un reste alkyle, alcoxy, alkylthio, alkylsulfinyle, alkylsulfonyle, halogéno-alkyle, halogéno-alcoxy, halogéno-alkylthio, halogéno-alkylsulfinyle, ou halogéno-alkylsulfonyle chaque fois linéaire ou ramifié et ayant chacun 1 à 4 atomes de carbone dans les diverses parties alkyles et portant éventuellement 1 à 9 atomes d'halogène identiques ou différents ;

Y représente O, S, SO ou $SO_2$, et

Ar représente un groupe phényle substitué deux ou plus de deux fois et un groupe 2-pyridyle, 3-pyridyle ou 4-pyridyle éventuellement substitué une ou plusieurs fois, et l'on peut citer comme substituant fixé sur le groupe phényle ou sur le groupe pyridyle : un reste cyano, nitro, halogéno, un reste alkyle, alcoxy ou alcoxycarbonyle linéaire ou ramifié ayant chaque fois jusqu'à 4 atomes de carbone et, en outre, un reste halogéno-alkyle ou halogéno-alcoxy, linéaire ou ramifié ayant chacun jusqu'à 4 atomes de carbone et portant jusqu'à 9 atomes d'halogène, identiques ou différents, ou bien un reste $-S-(O)_mR^6$,
dans lequel

$R^6$ représente un groupe amino, ainsi qu'un groupe alkyle, alkylamino, dialkylamino ou halogéno-alkyle linéaire ou ramifié, ayant chacun jusqu'à 4 atomes de carbone dans les parties alkyles individuelles et dans le cas du groupe halogéno-alkyle, ayant jusqu'à 9 atomes d'halogène identiques ou différents, et

m est un nombre valant 0, 1 ou 2.

**2.** Dérivés de 4-cyano(nitro)-5-oxy(thio)-pyrazole de formule (I) selon la revendication 1, dans laquelle :

$R^1$ représente un atome d'hydrogène, un groupe méthyle, éthyle, n-propyle ou isopropyle et trifluorométhyle ;

$R^2$ représente un groupe nitro ou cyano ;

$R^3$ représente un groupe méthyle, éthyle, n- ou i-propyle ; n-, i-, s- ou t-butyle; n- ou i-pentyle ; n- ou i-hexyle, allyle, propényle, butényle, propargyle ou butynyle, qui peuvent chacun être substitués une ou plusieurs fois, de façon identique ou différente, par un atome de fluor, de chlore ou de brome, par un reste méthoxy, éthoxy, méthylthio, éthylthio,

méthylsulfinyle, méthylsulfonyle, diméthoxy, diéthoxy, cyano, hydroxycarbonyle, méthoxycarbonyle, éthoxycarbonyle, propoxycarbonyle, butoxycarbonyle, aminocarbonyle, méthylaminocarbonyle, diméthylaminocarbonyle, méthylthiocarbonyle, méthylcarbonyle, hydroxyimino, méthoximino, éthoximino, phosphonoyle, méthyl-phosphinoyle, diméthylphosphinoyle, méthyl-éthylphosphinoyle, diméthylphosphonoyle,et diéthylphosphonoyle ; ainsi qu'un groupe cyclopropyle, cyclopentyle, cyclohexyle, cyclopropylméthyle, cyclopentylméthyle ou cyclohexylméthyle, qui peuvent chacun être substitués, une ou deux fois, de façon identique ou différente par un atome de fluor, de chlore ou par un groupe méthyle ;
ou un groupe phényle benzyle ou phényléthyle éventuellement substitué une à trois fois, de façon identique ou différente, et l'on peut citer comme substituant fixé sur le groupe phényle : un atome de fluor, de chlore, de brome d'iode, un reste cyano, nitro, méthyle, éthyle, méthoxy, méthylthio, trifluorométhyle, méthylsulfinyle, méthylsulfonyle, trifluorométhoxy, trifluorométhylthio, trifluorométhylsulfinyle et trifluorométhylsulfonyle ;

Y représente O, S, SO ou $SO_2$ et

Ar représente un groupe phényle substitué deux ou plus de deux fois ainsi qu'un groupe 2-pyridyle, 3-pyridyle ou 4-pyridyle chacun éventuellement substitué une à quatre fois de façon identique ou différente, et l'on peut citer comme substituant du groupe phényle ou du groupe pyridyle :
un reste cyano, nitro, fluoro, chloro, bromo, iode, méthyle, éthyle, n- et i-propyle, n-, i-, s- et t-butyle, méthoxy, éthoxy, méthoxycarbonyle, éthoxycarbonyle, trifluorométhyle, trichlorométhyle, difluorochlorométhyle, dichlorofluorométhyle, chlorométhyle, dichlorométhyle, difluorométhyle, pentafluoroéthyle, tétrafluoréthyle, trifluorochloréthyle, trifluoréthyle, difluorodichloréthyle, trifluorodichloréthyle, pentachloréthyle, trifluorométhoxy; trichlorométhoxy, dichlorofluorométhoxy, difluorochlorométhoxy, chlorométhoxy, dichlorométhoxy, difluorométhoxy, pentafluoréthoxy, tétrafluoréthoxy, trifluorochloréthoxy, trifluoréthoxy, difluorodichloréthoxy, trifluorodichloréthoxy, pentachloréthoxy ou un reste $-S(O)_mR^6$,
dans laquelle

$R^6$ représente un groupe amino, méthylamino, éthylamino, diméthylamino, diéthylamino, fluorodichlorométhyle, difluorométhyle, tétrafluoroéthyle, trifluorochloréthyle, trifluorométhyle, méthyle ou éthyle et

m est un nombre valant 0, 1 ou 2.

**3.** Procédé pour préparer des dérivés de 4-cyano(nitro)-5-oxy(thio)-pyrazole de formule (I) :

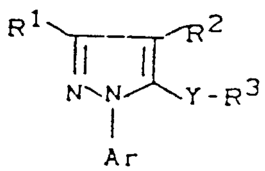

(I)

dans laquelle :

$R^1$ représente un atome d'hydrogène, un groupe alkyle linéaire ou ramifié ayant 1 à 4 atomes de carbone ou un groupe halogénoalkyle, linéaire ou ramifié, ayant 1 à 4 atomes de carbone et 1 à 9 atomes d'halogène, identiques ou différents,

$R^2$ représente un groupe nitro ou cyano,

$R^3$ représente un groupe alkyle, alcényle ou alcynyle, linéaires ou ramifiés, chacun éventuellement substitué, et comportant chacun jusqu'à 4 atomes de carbone, et l'on peut alors citer comme substituants :
un atome d'halogène ; un reste alcoxy, alkythio, alkylsulfinyle, alkylsulfonyle et dialcoxy ayant chacun 1 à 4 atomes d'halogène dans la partie alkyle ; un reste cyano ; hydroxycarbonyle ; alcoxycarbonyle, alkylthiocarbonyle et alkylcarbonyle ayant chacun 1 à 4 atomes de carbone dans la partie alkyle ; aminocarbonyle ; alkylaminocarbonyle et dialkylaminocarbonyle ayant chacun 1 à 4 atomes de carbone dans les parties alkyles ; hydroxyimino et alcoxyimino ayant 1 à 4 atomes de carbone,
ainsi que le groupe

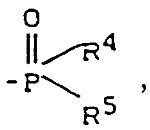

dans lequel

$R^4$ et $R^5$ sont identiques ou différents et représentent chacun un groupe hydroxy, alkyle et alcoxy ayant chacun 1 à 4 atomes de carbone, et un groupe phényle et phénoxy, éventuellement substitué une ou plusieurs fois, de façon identique ou différent, et l'on peut citer comme substituant, avantageusement les restes indiqués pour les substituants du groupe phényle ;

$R^3$ peut représenter en outre un groupe cycloalkyle et cycloalkylalkyle, identiques ou différents et chaque fois éventuellement substitués une ou plusieurs fois, le groupes ayant 3 à 7 atomes de carbone dans la partie cycloalkyle et 1 à 2 atomes de carbone dans la partie alkyle, et l'on peut citer comme substituant de la partie cycloalkyle : un atome d'halogène, un reste alkyle, alcoxy et alkylthio ayant chacun 1 à 4 atomes de carbone ;
ou un groupe phényle et phénylalkyle, éventuellement substitué une ou plusieurs de façon identique ou différente et comportant 1 à 4 atomes de carbone dans la partie alkyle linéaire ou ramifiée, et l'on peut citer comme substituant du noyau phényle, à chaque fois : un atome d'halogène, un reste cyano, nitro, un reste alkyle, alcoxy, alkylthio, alkylsulfinyle, alkylsulfonyle, halogéno-alkyle, halogéno-alcoxy, halogéno-alkylthio, halogéno-alkylsulfinyle, ou halogéno-alkylsulfonyle chaque fois linéaire ou ramifié et ayant chacun 1 à 4 atomes de carbone dans les diverses parties alkyles et portant éventuellement 1 à 9 atomes d'halogène identiques ou différents ;

Y représente O, S, SO ou $SO_2$, et

Ar représente un groupe phényle substitué deux ou plus de deux fois et un groupe 2-pyridyle, 3-pyridyle ou 4-pyridyle éventuellement substitué une ou plusieurs fois, et l'on peut citer comme substituant fixé sur le groupe phényle ou sur le groupe pyridyle : un reste cyano, nitro, halogéno, un reste alkyle, alcoxy ou alcoxycarbonyle linéaire ou ramifié ayant chaque fois jusqu'à 4 atomes de carbone et, en outre, un reste halogéno-alkyle ou halogéno-alcoxy, linéaire ou ramifié ayant chacun jusqu'à 4 atomes de carbone et portant jusqu'à 9 atomes d'halogène, identiques ou différents, ou bien un reste $-S-(O)_mR^6$,
dans lequel

$R^6$ représente un groupe amino, ainsi qu'un groupe alkyle, alkylamino, dialkylamino ou halogéno-alkyle linéaire ou ramifié, ayant chacun jusqu'à 4 atomes de carbone dans les parties alkyles individuelles et dans le cas du groupe halogéno-alkyle, ayant jusqu'à 9 atomes d'halogène identiques ou différents, et

m est un nombre valant 0, 1 ou 2,

caractérisé en ce que

a) on obtient des dérivés de 4-cyano(nitro)-5-oxy(thio)-pyrazole, de formule (I):

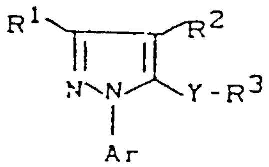

(I)

dans laquelle
$R^1$, $R^2$, $R^3$, Y et Ar ont le sens indiqué ci-dessus,
en faisant réagir, en présence d'un diluant et éventuellement en présence d'un agent de fixation des acides, des dérivés de 5-halogéno-pyrazole de formule (II) :

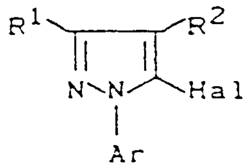

(II)

dans laquelle
$R^1$, $R^2$ et Ar ont le sens indiqué, et
Hal représente un atome de fluor, de chlore ou de brome, avec des composés nucléophiles de formule (III) :

$H - Y - R^3$ (III)

dans laquelle
$R^3$ et Y ont le sens indiqué ci-dessus, ou bien
b) on obtient des dérivés de 4-nitro-5-oxy(thio)-pyrazole de formule (Ia):

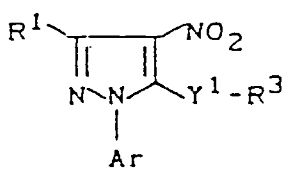

(Ia)

dans laquelle
$R^1$, $R^3$ et Ar ont le sens indiqué ci-dessus, et
$Y^1$ représente un atome d'oxygène ou de soufre,
en faisant réagir des dérivés de 5-oxy(thio)-pyrazoles de formule (IV) :

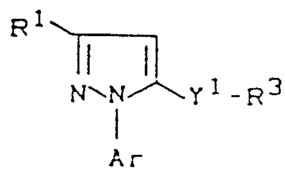

(IV)

dans laquelle
$R^1$, $R^3$, $Y^1$ et Ar ont le sens indiqué ci-dessus,
avec un agent de nitration, en opérant éventuellement en présence d'un diluant et éventuellement en présence d'un catalyseur, ou bien
c) on obtient des dérivés de 4-cyano(nitro)-5-oxy(thio)-pyrazoles de formule (Ib) :

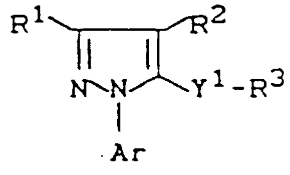

(Ib)

dans laquelle
$R^1$, $R^2$, $R^3$, $Y^1$ et Ar ont le sens indiqué ci-dessus, en faisant réagir des pyrazoline-ones (thiones) de formule (V) :

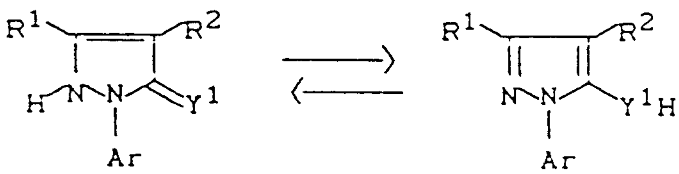

(V)

dans lesquelles
$R^1$, $R^2$, Ar et $Y^1$ ont le sens indiqué ci-dessus,
avec des-agents d'alkylation de formule (VI) :

A - $R^3$ (VI)

dans laquelle
$R^3$ a le sens indiqué ci-dessus, et
A représente un groupe partant capable d'enlever des électrons,
en opérant éventuellement en présence d'un diluant et éventuellement en présence d'un agent de fixation des acides et, ou bien
d) on obtient des dérivés de 5-sulfinyl(sulfonyl)-pyrazoles de formule (Ic) :

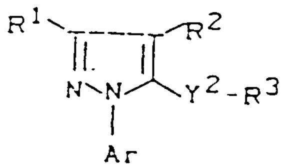

(Ic)

dans laquelle
$R^1$, $R^2$, $R^3$ et Ar ont le sens indiqué ci-dessus, et
$Y^2$ représente SO ou $SO_2$,
en soumettant des dérivés de 5-thio-pyrazoles de formule (Id) :

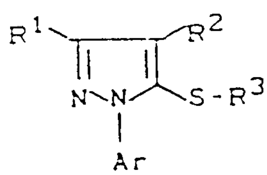

(Id)

dans laquelle
$R^1$, $R^2$, $R^3$ et Ar ont le sens indiqué ci-dessus,
à une oxydation, ou bien
e) on obtient des dérivés de 4-cyano(nitro)-5-oxy(thio)-pyrazoles de formule (Ib) :

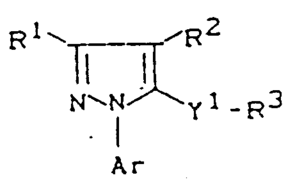

(Ib)

dans laquelle
$R^1$, $R^2$, $R^3$, $Y^1$ et Ar ont le sens indiqué ci-dessus,
en faisant réagir des dérivés de 5-sulfonyl-pyrazoles de formule (Ie) :

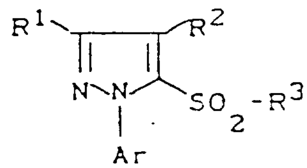

(Ie)

dans laquelle
$R^1$, $R^2$, $R^3$ et Ar ont le sens indiqué ci-dessus,
avec des composés nucléophiles de formule (IIIa) :

$H - Y^1 - R^3$ (IIIa)

dans laquelle
$R^3$ et $Y^1$ ont le sens indiqué ci-dessus,
en opérant en présence d'un diluant et éventuellement en présence d'un agent de fixation des acides.

4. Herbicides et agents de régulation de la croissance des plantes, caractérisés en ce qu'ils contiennent au moins un dérivé de 4-cyano(nitro)-5-oxy(thio)-pyrazole de formule (I) selon les revendications 1 à 3.

5. Procédé pour lutter contres des mauvaises herbes, caractérisé en ce qu'on laisse agir, sur les mauvaises herbes et/ou sur leur biotope ou espace vital, des dérivés de 4-cyano(nitro)-5-oxy-(thio)-pyrazole de formule (I) selon les revendications 1 à 3.

6. Utilisation de dérivés de 4-cyano(nitro)-5-oxy(thio)-pyrazole de formule (I) selon les revendications 1 à 3 pour lutter contre des mauvaises herbes et/ou pour servir de régulateurs de la croissance des plantes.

7. Procédé pour produire des herbicides et/ou des agents de régulation de la croissance des plantes, caractérisé en ce qu'on mélange des dérivés de 4-cyano(nitro)-5-oxy(thio)-pyrazole de formule (I) selon les revendications 1 à 3 avec des agents d'allongement et/ou avec des substances tensioactives.

8. Dérivés de 5-oxy(thio)-pyrazole de formule (IV) :

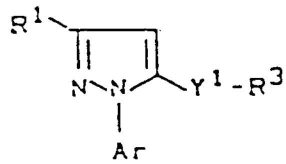

(IV)

dans laquelle

$R^1$ représente un atome d'hydrogène, un groupe alkyle linéaire ou ramifié ayant 1 à 4 atomes de carbone ou un groupe halogénoalkyle, linéaire ou ramifié, ayant 1 à 4 atomes de carbone et comportant 1 à 9 atomes d'halogène, identiques ou différents,

$R^3$ représente un groupe alkyle, alcényle ou alcynyle linéaire ou ramifié, chacun éventuellement substitué, ayant jusqu'à 6 atomes de carbone, et l'on peut citer comme substituants à chaque fois:
un atome d'halogène ; un reste alcoxy, alkythio, alkylsulfinyle, alkylsulfonyle et dialcoxy ayant à chacun 1 à 4 atomes de carbone dans les parties alkyles ; un reste cyano ; hydroxycarbonyle ; alcoxycarbonyle, alkylthiocarbonyle et alkylcarbonyle ayant chacun 1 à 4 atomes de carbone dans la partie alkyle ; aminocarbonyle ; alkylaminocarbonyle et dialkylaminocarbonyle ayant chacun 1 à 4 atomes de carbone dans les parties alkyles ; hydroxyimino et alcoxyimino ayant 1 à 4 atomes de carbone ;
ainsi que le groupement

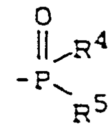 ,

dans lequel

$R^4$ et $R^5$ sont identiques ou différents et représentent chacun un groupe hydroxy, alkyle et alcoxy ayant chacun 1 à 4 atomes de carbone, ainsi qu'un groupe phényle et phénoxy, éventuellement substitué une ou plusieurs fois, de façon identique ou différente, et l'on peut citer comme substituant, avantageusement les restes indiqués comme substituant du groupe phényle Ar ;
ainsi qu'un groupe cycloalkyle et cycloalkylalkyle, chacun éventuellement substitué une ou plusieurs fois de façon identique ou différente et comporant 3 à 7 atomes de carbone dans la partie cycloalkyle et 1 à 2 atomes de carbone dans la partie alkyle, et l'on peut citer comme substituant de la partie cycloalkyle : un atome d'halogène, un reste alkyle, alcoxy et alkylthio ayant chacun 1 à 4 atomes de carbone ;
ou un groupe phényle ou phénylalkyle, chacun éventuellement substitué une ou plusieurs fois de façon identique ou différente et ayant 1 à 4 atomes de carbone dans la partie alkyle linéaire ou ramifiée, et l'on peut citer à chaque fois comme substituant du groupe phényle : un reste halogéno, cyano, nitro, un reste alkyle, alcoxy, alkylthio, alkylsulfinyle, alkylsulfonyle, halogéno-alkyle, halogéno-alcoxy, halogéno-alkylthio, halogéno-alkylsulfinyle, ou halogéno-alkylsulfonyle ayant chacun 1 à 4 atomes de carbone dans les parties alkyles individuelles et comporant éventuellement 1 à 9 atomes d'halogène identiques ou différents,

$Y^1$ représente un atome d'oxygène ou de soufre, et

Ar représente un groupe phényle substitué deux ou plus de deux fois, ainsi qu'un groupe 2-pyridyle, 3-pyridyle ou 4-pyridyle éventuellement substitué une ou plusieurs fois, de façon identique ou différente, et l'on peut citer comme substituant du groupe phényle ou pyridyle : un reste cyano, nitro, halogéno, un reste alkyle, alcoxy ou alcoxycarbonyle linéaire ou ramifié ayant chacun jusqu'à 4 atomes de carbone et également un groupe halogéno-alkyle ou halogéno-alcoxy, linéaire ou ramifié ayant chacun jusqu'à 4 atomes de carbone et comportant jusqu'à 9 atomes d'halogène, identiques ou différents, ou bien un reste $-S-(O)_mR^6$,
dans lequel

$R^6$ représente un groupe amino, ainsi qu'un groupe alkyle, alkylamino, dialkylamino ou halogéno-alkyle linéaire ou ramifié et ayant chacun jusqu'à 4 atomes de carbone dans les parties alkyles individuelles, et dans le cas du groupe halogéno-alkyle, ayant jusqu'à 9 atomes d'halogène identiques ou différents, et

m est un nombre valant 0, 1 ou 2.

**9.** Pyrazoline-ones (thiones) de formule (V) :

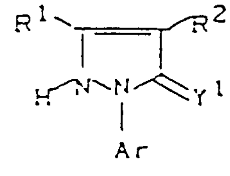 (V)

dans laquelle

$R^1$ représente un atome d'hydrogène, un groupe alkyle linéaire ou ramifié ayant 1 à 4 atomes de carbone ou un groupe halogéno-alkyle linéaire ou ramifié ayant 1 à 4 atomes de carbone et comportant 1 à 9 atomes d'halogène identiques ou différents,

$R^2$ représente un groupe nitro ou cyano ;

$Y^1$ représente un atome d'hydrogène ou de soufre, et

Ar représente un groupe phényle substitué deux ou plus de deux fois ainsi qu'un groupe 2-pyridyle, 3-pyridyle ou 4-pyridyle, éventuellement substitué une ou plusieurs fois de façon

identique ou différente, et l'on peut citer comme substituant du noyau phényle ou du noyau pyridyle :
un reste cyano, nitro, halogéno, un reste alkyle, alcoxy ou alcoxycarbonyle linéaire ou ramifié et ayant chacun jusqu'à 4 atomes de carbone, ainsi qu'un reste halogéno-alkyle ou halogéno-alcoxy linéaire ou ramifié ayant chacun jusqu'à 4 atomes de carbone et jusqu'à 9 atomes d'halogène identiques ou différents, ou un reste $-S(O)_mR^6$,
dans lequel

$R^6$ représente un groupe amino, ainsi qu'un groupe alkyle, alkylamino, dialkyl-amino ou halogéno-alkyle linéaire ou ramifié ayant chacun jusqu'à 4 atomes de carbone dans les diverses parties alkyles et, dans le cas du groupe halogéno-alkyle, ayant jusqu'à 9 atomes d'halogène identiques ou différents, et

m est un nombre valant 0, 1 ou 2.